(19) **Europäisches Patentamt / European Patent Office / Office européen des brevets**

(11) **EP 3 384 045 B1**

(12) **EUROPEAN PATENT SPECIFICATION**

(45) Date of publication and mention
of the grant of the patent:
**20.01.2021 Bulletin 2021/03**

(21) Application number: **16809319.3**

(22) Date of filing: **01.12.2016**

(51) Int Cl.:
***C12Q 1/68*** (2018.01)

(86) International application number:
**PCT/EP2016/079427**

(87) International publication number:
**WO 2017/093400 (08.06.2017 Gazette 2017/23)**

(54) **METHOD FOR DETERMINING CELL CLONALITY**

VERFAHREN ZUR BESTIMMUNG VON ZELLOBERFLÄCHEN-ANTIGENKLONALITÄT

PROCÉDÉ PERMETTANT DE DÉTERMINER UNE CLONALITÉ DE CELLULE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **03.12.2015 EP 15197894**

(43) Date of publication of application:
**10.10.2018 Bulletin 2018/41**

(73) Proprietor: **Ares Trading S.A.
1170 Aubonne (CH)**

(72) Inventors:
• **LA NEVE, Fabio
10138 Torino (IT)**
• **FEGER, Georg
1205 Geneva (CH)**
• **TOSO, Emiliano
13888 Mongrando (BI) (IT)**

(74) Representative: **Merck Serono S.A.
Intellectual Property
Terre Bonne Business Park
Building Z0
Route de Crassier 1
1262 Eysins (CH)**

(56) References cited:
WO-A2-2012/083069    KR-A- 20140 096 988
US-A1- 2013 130 311    US-A1- 2015 038 337

• SUFFICOOL KARI E ET AL: "T-cell clonality assessment by next-generation sequencing improves detection sensitivity in mycosis fungoides.", August 2015 (2015-08), JOURNAL OF THE AMERICAN ACADEMY OF DERMATOLOGY AUG 2015, VOL. 73, NR. 2, PAGE(S) 228 - 36.E2, XP002766636, ISSN: 1097-6787 figure 2
• BLECK G T: "An Alternative Method for the Rapid Generation of Stable, High-Expressing Mammalian Cell Lines", BioProcessing Journal , vol. 1, no. 7 1 September 2005 (2005-09-01), XP002761142, Retrieved from the Internet: URL:http://www.gala.com/pdf/bioprocessingj .pdf [retrieved on 2016-09-02]
• BOYD S D ET AL: "Measurement and clinical monitoring of human lymphocyte clonality by massively parallel V-D-J pyrosequencing", SCIENCE TRANSLATIONAL MEDICINE, AMERICAN ASSOCIATION FOR THE ADVANCEMENT OF SCIENCE, WASHINGTON, DC, vol. 1, no. 12, 23 December 2009 (2009-12-23), pages 1-8, XP003031087, ISSN: 1946-6234

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**BACKGROUND**

Recombinant mammalian cell lines are a powerful tool for the production of therapeutic proteins due to their capacity to properly fold and assemble proteins and add post translational modifications to complex proteins similar to those found in humans. In fact most, cell lines used for biopharmaceutical protein production to date have originated from mammals through various ways of immortalization. Today about 60-70% of all recombinant protein pharmaceuticals are produced in mammalian cells. In addition, several hundred clinical candidate therapeutic proteins are in current company pipelines. Many of these proteins are expressed in immortalized Chinese hamster ovary (CHO) cells, but other cell lines, such as those derived from mouse myeloma (NS0, SP2/0), baby hamster kidney (BHK), human embryo kidney (HEK-293) and human retinal cells have gained regulatory approval as a common tool for protein production in the pharmaceutical industry.

The first step in the manufacturing of a biopharmaceutical protein in a mammalian cell system is the generation of a stable, monoclonal cell line that has stably integrated the transgene encoding the protein of interest in its genome. Several transfection methods, like calcium phosphate precipitation, electroporation, lipofectamine and viral transfection are commonly used in order to deliver the transgene into the host cell nucleus for chromosomal integration. One of the most commonly used transfection method in clinical research is virus-mediated transfection, also known as transduction. This technology has also been used to generate recombinant cell lines for the manufacturing of therapeutic proteins. Virus-mediated transfection is highly efficient and it is easy to achieve sustainable transgene expression. Once the DNA enters the nucleus, the transgene is integrated into the host cell genome and expression of the gene of interest (GOI) that it contains is, in part, dictated by the surrounding chromosomal structure and associated features. However, one major drawback of viral transfection is the unpredictable insertion of the transgene into the host cell genome. Such insertion has the effect that both protein quality and quantity become highly dependent on the genomic location of the protein-encoding transgene.

For the generation of the protein-producing cell line a selection marker is co-transfected with the GOI into the host cell genome. Positively transfected cells are then selected by cultivation in the appropriate selection medium. The most common genes for selection are dihydrofolate reductase (DHFR), an enzyme involved in nucleotide metabolism, and glutamine synthetase (GS). In both cases, selection occurs in the absence of the appropriate metabolite, preventing growth of the non-transfected cells. Usually, the transfected cells are first screened for proliferation and protein expression to identify the candidates with the best production potential and growth characteristics. In the next step a pool of cells is isolated and enriched for clones with highly specific productivity. Sufficool et al. (J. Am. Acad. Derm. Aug 2015, Vol. 73, Nr.2, pages 228-36.E2) describe a method for determining whether in a pool of T-cells present in a tissue sample clonal expansion of T-cells has taken place. This method relies on the fact that if clonal expansion of the T-cell took place in the tissue, there is an unusual high copy number of the unique TCR sequence from that particular T-cell present in the pool of all variants of TCR sequences in said tissue. The method of Sufficool et al. determines the relative amounts of unique TCR gene sequences. This pool, however, is heterogeneous; it comprises cells with different transgene insertion site(s) and copy numbers in their genomes, resulting in variation of the protein expression level.

A critical component in controlling the production of biological products from mammalian cell lines is the characterization and testing of the cell substrate in order to ensure identity, purity and suitability of these cells for the manufacturing process. Therefore, one needs to isolate clones derived from single cells (ICH Q5D) exhibiting the highest protein expression levels, proliferation rates, and the best product quality. To this end, single cells are recovered from the heterogeneous cell pool by a series of limited dilution steps or single cell deposition by FACS with concomitant imaging and elaborate screening to isolate a small number of candidate clones. The most promising candidates are then cryopreserved as the pre-master cell bank (pre-MCB) and are evaluated for passaging stability that should cover the longest possible manufacturing duration. In addition to phenotypic stability, the cell lines need to be evaluated for genetic stability in the absence of selective pressure, which generally is not applied at the production stage. Once an optimal cell line has been identified, a master cell bank (MCB) is produced. An MCB is defined as an aliquot of a single pool of cells that has been prepared from a single selected cell clone under defined conditions, dispensed into multiple vials, and stored under defined conditions (usually -100°C and below). Process development for commercial production is initiated based on such an MCB, that is expanded to generate the working cell banks (WCBs), which is used for the manufacturing process. The approval of an MCB and WCB as a production cell line in pharmaceutical industry depends on the fulfillment of strict requirements imposed by the national and international health authorities. Important requirements for the approval of recombinant cell lines as a production cell line include the validation of clonality and testing for microbial contaminations of the cell line (e.g. retroviruses or mycoplasma).

In case of lack of documented evidence of clonality which could be due to single limited round of dilution cloning instead of two rounds, lack of imaging on day one in case of FACS cloning or incomplete documentation additional evidence will be required by the health authorities to increase the assurance of clonality of the MCB. The most accepted methods are FISH and Southern blot analysis of the MCB and WCB. However, this methods lacks often sensitivity especially in cell lines with low copy number of the GOI and is therefore often difficult to interpret. In addition, all cell lines with more than a few insertion sites such as those generated by viral transduction containing tens or even hundreds of insertion sites cannot be analyzed by FISH. Southern blot analyzes can also be used but is lacking sensitivity in case if there is a very low percentage of contamination and unless sufficient number of restriction enzymes are used. It requires the knowledge genomic regions flanking the GOI insertion site. Additional supportive evidence could be derived from the monitoring of protein production titers and various specific characteristics including cell morphology, cell culture parameter including cell viability, cell growth and product quality. Such procedures, however, are highly labor-intensive and time-consuming. There thus exists a strong motivation to provide new ways of determining the clonality of production cell lines (MCB) to circumvent the laborious, expensive and slow screening of clones currently necessary before a given cell line can be approved for the use in the production of a pharmaceutical protein. Providing a method to determine the clonality of a given MCB by means that greatly reduce the time-consuming and labor-intensive evaluation steps would greatly assist promoting fast and reliable quality assessment of the clonality of the production cell lines. It is an aim of the present invention to address such needs.

## SUMMARY OF THE INVENTION

In accordance with the above aim, the present disclosure relates to a method for determining the clonality of a Master Cell Bank (MCB), said MCB resulting from predictable or not predictable insertion of a transgene of known sequence into a host progenitor cell (HPC) genome of known sequence.

In one aspect of the present disclosure the method comprises the steps of:

> a) Identifying one or more transgene insertion regions (TIRs) in the genome of a reference subclone cell (RSC), wherein the RSC has been expanded from the MCB for which clonality is to be determined, and wherein said identifying is achieved by
>
> > i. paired-end sequencing of said RSC genome to obtain an RSC genome sequence or RSC genome sequences; and
> > ii. alignment of said RSC genome sequence or sequences to said known HPC genome sequence and said known transgene sequence,
>
> thereby yielding one or more transgene insertion regions (TIRs);
> b) Determining one or more TIRs as identified in step (a) with the highest degree of sequence coverage,
>
> > wherein said sequence coverage refers to the number of times a given nucleic acid sequence containing a given TIR is read during the sequencing process by partially overlapping reads;
> > wherein said one or more TIRs with the highest degree of sequence coverage are assigned as reference TIRs (RTIRs);
>
> c) Identifying one or more transgene insertion regions (TIRs) in the respective genomes of one or more subclone cells (SCs);
>
> > wherein each of the SCs has been expanded from the MCB for which clonality is to be determined but is independent of said RSC,
> > wherein said identifying is achieved by
> >
> > > i. paired-end sequencing of each respective SC genome to obtain an SC genome sequence or SC genome sequences; and
> > > ii. alignment of each respective SC genome sequence or sequences to said known HPC genome sequence and said known transgene sequence,
>
> thereby yielding one or more comparative transgene insertion regions (CTIRs);
> d) Comparing said one or more RTIRs determined in step (b) with the respective CTIRs determined in step (c);
> e) Evaluating the correspondence between each of said one or more CTIRs present in a respective SC and corresponding RTIRs present in said RSC; and
> f) Determining clonality of said MCB based on said correspondence evaluated in part (e). In an alternative aspect of the present the method comprises the steps of:
> g) Identifying one or more transgene insertion regions (TIRs) in the genome of a reference subclone cell (RSC), wherein the RSC has been expanded from the MCB for which clonality is to be determined, and wherein said identifying is achieved by

iii. paired-end sequencing of said RSC genome to obtain an RSC genome sequence or RSC genome sequences; and
iv. alignment of said RSC genome sequence or sequences to said known
HPC genome sequence and said known transgene sequence, thereby yielding one or more transgene insertion regions (TIRs);

h) Determining one or more TIRs as identified in step (a) with the highest degree of sequence coverage,

wherein said sequence coverage refers to the number of times a given nucleic acid sequence containing a given TIR is read during the sequencing process by partially overlapping reads;
wherein said one or more TIRs with the highest degree of sequence coverage are assigned as reference TIRs (RTIRs);

i) Identifying one or more transgene insertion regions (TIRs) in the respective genomes of one or more subclone cells (SCs);

wherein each of the SCs has been expanded from the MCB for which clonality is to be determined but is independent of said RSC,
wherein said identifying is achieved by

iii. paired-end sequencing of each respective SC genome to obtain an SC genome sequence or SC genome sequences; and
iv. alignment of each respective SC genome sequence or sequences to said known HPC genome sequence and said known transgene sequence,

thereby yielding one or more comparative transgene insertion regions (CTIRs);
j) Comparing said one or more RTIRs determined in step (b) with the respective CTIRs determined in step (c);
k) Evaluating the correspondence between each of said one or more CTIRs present in a respective SC and corresponding RTIRs present in said RSC; and
l) Determining clonality of said MCB based on said correspondence evaluated in part (e),

wherein said MCB is considered to be monoclonal, if said RSC and said one or more SCs are grouped into the same cluster.

As explained above, monoclonality of MCBs is traditionally achieved by limited dilution of a pool of cells into which a transgene has been inserted to yield one single cell per well of a multi-well plate. Because the transgene insertion in each individual target cell is different (random or quasi random), one expects that one MCB cell in one well will differ from another MCB cell in another well, as each MCB cell represents the result of independent transgene insertion at different locations within the cell's genome. This means that, following transgene insertion into a previously homogeneous population of host progenitor cells, one obtains a transgenically heterogeneous mixture of different cells which, following dilution, are isolated, separately from transgenically distinct cells, in a single well of a multi-well plate.

Such isolation by dilution is however based on a calculated statistical probability. Thus, while most wells of a multi-well plate will likely contain only one MCB cell, it cannot be excluded that some wells may contain more than one MCB cell, while other wells might contain none. Especially the former scenario, in which a single well contains multiple transgenically heterogeneous cells, which would give rise to a heterogeneous MCBs, will complicate and even jeopardize the regulatory approval process for a pharmaceutical protein resulting from expression of an inserted transgene. This is because in such a scenario one would incorrectly assume (based on the statistical calculation of the dilution factor) that the protein in question is being expressed from only a single type of cell, with a uniform transgene insertion profile, when in fact the protein is being expressed from multiple types of cells, each with a distinct transgene insertion profile. Such variation may lead to differences in the nature of the protein produced, the exclusion of which is a prerequisite in the regulatory approval process. Therefore usually two rounds of limited dilution cloning are applied to minimize the chance of having more then on MCB cell per well. Alternatively a single round of limited dilution or single cell deposition can be monitored by imaging confirming that a single MCB cell has been put into a single well. However, none of these methods can guarantee the fact that a single cell has been deposited into the well. In the case of limited dilution there is always a statistical uncertainty albeit small in the case of two rounds of limited dilution cloning. In the case cell imaging a second cell might not be detected because it is in a corner of the well, attached to the side of the well or otherwise being out of focal plane of the camera.

By comparing selected transgene insertion sites between a randomly chosen reference cell expanded from the (supposedly monoclonal) MCB with the corresponding insertion sites in other cells independently expanded from the (supposedly monoclonal) MCB, the above method advantageously allows reliable determination of monoclonality. In the event that all cells expanded from a given, supposedly monoclonal MCB exhibit identical transgene insertion sites, then one may reliably conclude that the MCB is in fact monoclonal. However, in the event that cells expanded from a given, supposedly monoclonal MCB exhibit divergent transgene insertion profiles, then one may reliably conclude that the supposedly monoclonal MCB is in fact not monoclonal, and instead results from transgene insertion at incongruent genomic locations. Such reliable information is invaluable in the regulatory approval process for recombinantly produced proteins intended for pharmaceutical application.

In one embodiment of the invention, the paired-end sequencing involves sequencing of a given nucleic acid molecule from both ends of said nucleic acid molecule, thereby generating pairs of reads for a given nucleic acid molecule representing a fragment of the genome to be sequenced. In a further embodiment of the invention, the RSC is sequenced with a higher sequence coverage compared to said one or more SCs.

In a further embodiment of the invention said MCB results from the insertion of said transgene at a multiple positions into said HPC genome, wherein said insertion is preferably effected using a retroviral vector.

In a further embodiment of the invention, the determination of TIRs comprises classification of paired-end "read 1" sequences and paired-end "read 2" sequences derived from the paired-end libraries into 4 classes,

wherein class 1 comprises "read 1" sequences mapping to said transgene; class 2 comprises "read 1" sequences mapping to said HPC genome; class 3 comprises "read 2" sequences mapping to said transgene; and class 4 comprises "read 2" sequences mapping to said HPC genome,

wherein said "read 1" and said "read 2" represent respective forward and backward reads corresponding to the 5' and 3' ends of a given nucleic acid molecule within a nucleic acid cluster generated in sequencing of a nucleic acid library of said RSC or said one or more SCs.

In a further embodiment of the invention, the determination of TIRs comprises classification of paired-end "read 1" sequences and paired-end "read 2" sequences derived from the paired-end libraries into 4 classes,

wherein class 1 comprises "read 1" sequences mapping exclusively to said transgene; class 2 comprises "read 1" sequences mapping exclusively to said HPC genome; class 3 comprises "read 2" sequences mapping exclusively to said transgene; and class 4 comprises "read 2" sequences mapping exclusively to said HPC genome,

wherein said "read 1" and said "read 2" represent respective forward and backward reads corresponding to the 5' and 3' ends of a given nucleic acid molecule within a nucleic acid cluster generated in sequencing of a nucleic acid library of said RSC or said one or more SCs.

In a further embodiment of the invention, "read 1" sequences are combined with the corresponding, "read 2" sequences using a flow cell sequence identifier,

wherein said sequence identifier comprises information of the flow cell lane, the tile number within the flow cell, the "x" coordinate of the nucleic acid cluster within a tile, and the "y" coordinate of the nucleic acid cluster within a tile, thereby assigning each sequence pair corresponding to "read 1" and "read 2" sequences a unique position within the flow cell.

In a further embodiment of the invention, the respective "read 1" and "read 2" sequences of a respective read pair are separately aligned to the known sequences of the transgene and the HPC genome.

In a further embodiment of the invention, only the read pairs comprising class 1 and 4 sequences and the read pairs comprising class 2 and class 3 sequences are selected for further analysis.

In a further embodiment of the invention, said TIRs are identified by aligning the paired-end read sequences corresponding to class 2 and class 4 to the HPC genome, thereby defining a 2kb region for each of said TIRs in the HPC genome.

In a further embodiment of the invention, the method for determining clonality of a MCB comprises determining n RTIRs with the highest sequence coverage in the paired-end NGS library, wherein n is an integer from 5 to 50. For example, the integer and may be set as 5, 10, 15, 20, 25, 30, 35, 40, 45 or 50.

In a further embodiment of the invention, the first n RTIRs with highest sequence coverage are determined based on (a) the number of reads of a respective paired-end sequence corresponding to class 2 and class 4 mapping to the HPC genome, wherein higher number of reads indicates inclusion as an RTIR, and (b) the partial overlap of the number of reads of a respective paired-end read sequence corresponding to class 2 and 4, wherein lower partial overlap of number of reads indicates inclusion as an RTIR.

In a further embodiment of the invention, each of the first n RTIRs in said RSC genome is compared with the corresponding genomic location of said CTIRs in each of said one or more SC genomes. In a further embodiment of the invention, comparison of said RTIRs in said RSC and said CTIRs in said one or more SCs is achieved by generating a presence/absence matrix of insertion regions, wherein one matrix dimension represents said n RTIRs of said transgene in said RSC genome and another, preferably orthogonal, matrix dimension represents said RSC and each of said one or more SCs.

In a further embodiment of the invention, the presence or absence of a respective CTIR in said one or more SCs relative to a respective RTIR in said RSC is represented in the matrix as a binary color code, wherein a first color represents the respective presence or absence of a respective RTIR in said RSC, the respective presence or absence of a respective CTIR in said one or more SCs; and wherein a second color represents the respective absence or presence of a respective RTIR in said RSC, the respective absence or presence of a respective CTIR in said one or more SCs.

In a further embodiment of the invention, the relationship between said RSC and each of the said one or more SCs is evaluated by calculation of a distance matrix.

In a further embodiment of the invention, the distance matrix is calculated based on the following formula (I),

$$D_d\,(RSC, SC_m) = 1 - (2*\,N_{(total)}\,/\,[N_{(CTIR)} + N_{(RTIR)}])$$

wherein $D_d\,(RSC, SC_m)$ represents the distance function between said RSC genome and a respective $SC_m$ genome, wherein $N_{(total)}$ is the number of insertion regions present both in said RSC genome and said $SC_m$ genome; $N_{(CTIR)}$ is the total number of insertion regions present in said $SC_m$ genome; and $N_{(RTIR)}$ is the total number of insertion regions present in said RSC genome; wherein $D_d\,(RSC, SC_m)$ represents the distance, on a scale of 0 to 1; wherein a distance of 0 represents clonal identity between said RSC and a respective $SC_m$, and 1 represents clonal difference.

In a further embodiment of the invention, the parameters $N_{(total)}$, $N_{(CTIR)}$ and/or $N_{(RTIR)}$ are calculated based on the presence/absence matrix of insertion regions.

In a further embodiment of the invention, the method comprises representing said one or more SCs relative to the RSC on a common distance matrix.

In further embodiment of the invention, two respective genomes are considered to belong to a common cluster if the distance between them as calculated according to Formula (I) is 0.

In a further embodiment of the invention, said MCB is considered to be monoclonal, if said RSC and said one or more SCs are grouped into the same cluster.

## DESCRIPTION OF THE FIGURES

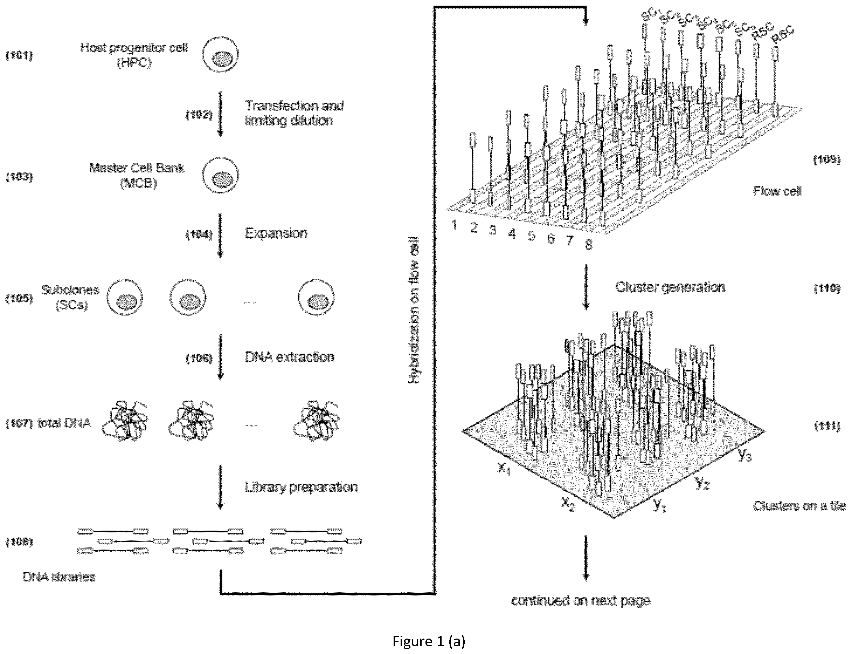

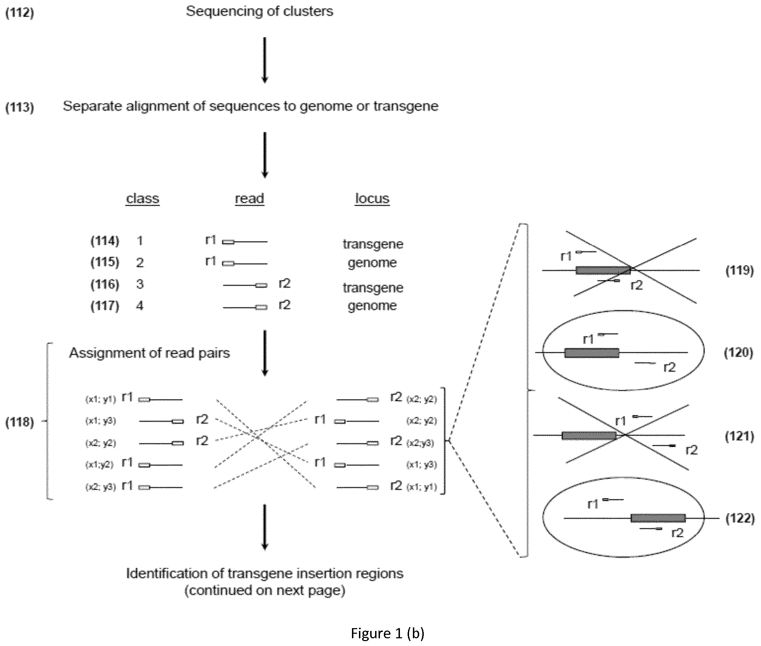

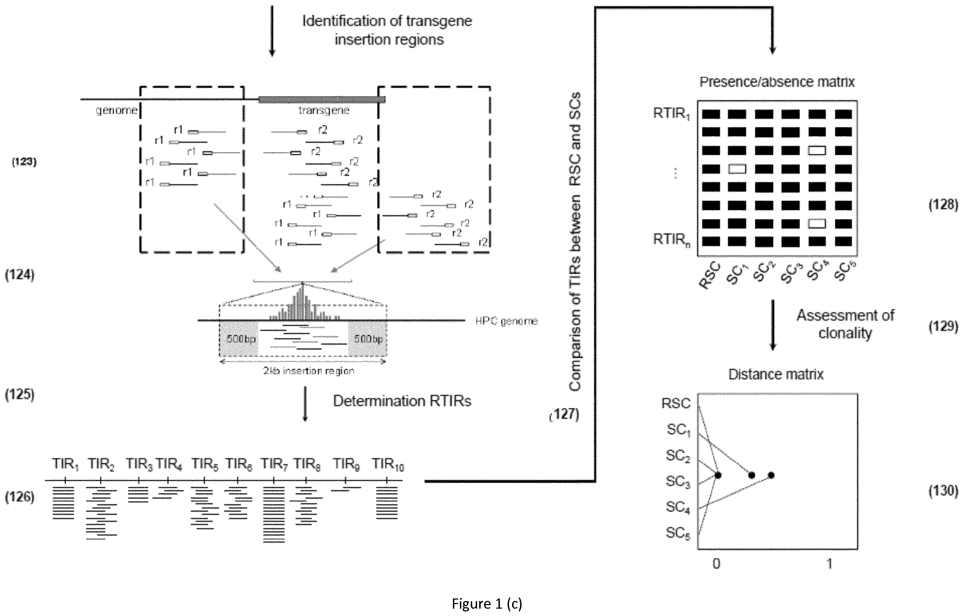

Figure 1 is a three-part continuous flow diagram illustrating one embodiment of the inventive method for determining the clonality of a master cell bank (MCB).

Figure 1(a): After transfection, for example retroviral-mediated transfection, of a host progenitor cell (HPC) (100) with a transgene and limiting dilution (101) (i.e. dilution to result in no more than one cell per intended aliquot volume), possible candidates for the MCB (102), containing the transgene at multiple positions in their respective genomes, are isolated and the individual cells are further expanded (103) to obtain one or more subclones (SCs) (104). In case of single cell analysis the individual cells are directly subjected to DNA extraction. Next, total DNA of the one or more subclones is extracted (105, 106) and converted into respective DNA libraries (107, 108) in preparation for sequencing (e.g. "next generation sequencing" (NGS)). As part of this preparation, each respective library may be hybridized on a flow cell (109) which is pre-prepared with addressed adapter sequences complementary to the genomic DNA fragments. Amplification and cluster generation (110) of the addressed templates then follows. This results in sequence clusters addressed on a respective tile of the flow cell (111), the addressing reflecting the original addressing of the complementary adapter sequences mentioned above.

Figure 1(b): Paired-end sequencing of the libraries is performed (112) and the obtained sequences are then aligned, for instance computer-aligned with sequences corresponding to either the HPC genome or transgene, both of which are known (113). The sequences are then conceptually separated into 4 classes as follows: class 1 corresponding to read 1 sequences aligning to the transgene (114); class 2 corresponding to read 1 sequences aligning to the HPC genome (115); class 3 corresponding to read 2 sequences aligning to the transgene (116); and class 4 corresponding to read 2 sequences aligning to the HPC genome (117). The read 1 and read 2 sequences represent respective forward and backward reads corresponding to the respective 5' and 3' ends of a given nucleic acid molecule within a given nucleic acid cluster (111) generated in sequencing of a given nucleic acid library (108).. Following the above sequence classification, read pairs are then assigned (118). Read 1 sequences of class 1 or 2 are combined with corresponding read 2 sequences of class 3 or 4. The correct assignment of read pairs belonging together may be achieved e.g. by a sequence identifier which is encoded in the FastQ file generated during the sequencing step for each nucleic acid molecule. The respective read 1 and read 2 sequences of a respective read pair are then separately aligned to the transgene sequence or to the HPC genome. Sequence pairs in which read 1 maps to the transgene (114) and read 2 maps to the HPC genome (117) (i.e. class 1/4 pairs (120)) and sequence pairs in which read 1 maps to the HPC genome (115) and read 2 maps to the transgene (116) (i.e. class 2/3 pairs (122)) are kept for further analysis. Sequence pairs in which read 1 and read 2 sequences both map either to the transgene (114, 116, 119) or to the HPC genome (115, 117, 121) are not suitable for the identification of transgene insertion region (TIRs) and are therefore discarded (119, 121) from the pool of sequence pairs. Sequence pairs that map to the HPC genome and that correspond to class 1/4 (114, 117, 120) and class 2/3 (115, 116, 122) read pairs comprise genome genomic sequences representing regions within the HPC genome that cover the boundaries between the HPC genome and the transgene.

Figure 1(c): TIRs are identified by alignment of paired end sequences corresponding to class 2 and class 4 to the HPC genome (123). For reasons of clarity, Figure 1(c) shows only the former case of alignment of class 2 sequences to the HPC genome, but one can imagine a mirror image case in which sequences of class 4 would align with the HPC genome on the opposite side of the inserted transgene. Alignment of reads to the HPC genome results in a window of approximately 1000 nucleotide (1 kb) for a transgene insertion region within the HPC genome. The window of approximately 1000 nucleotide is determined by the average insert size of the DNA library and can therefore be adjusted to specific needs. That is, the alignments described above and shown in Figure 1(c) (123) allows one to pinpoint the region of transgene insertion to within approximately 1 kb. To strengthen the statistical robustness of this prediction of TIR location, the determined TIR is extended to a 2 kb window, starting from the center of the 1 kb region and extending this 1 kb region on both sides by 500 bp (124). Next, the identified TIRs in a randomly picked subclone (RSC), which was sequenced with higher sequence coverage compared to the remaining subclones (SCs), are analyzed for their sequence coverage and the reads number to obtain one or more reference transgene insertion regions (RTIRs) (125). In particular, RTIRs are selected based on the total number of sequence reads that map to a given position on the HPC genome. TIRs that are represented by higher sequence coverage than others are assigned as RTIRs. A further requirement for the assignment as an RTIR is a low degree of overlap between the respective sequence reads that represents a given TIR. TIRs where sequence reads have a low degree of overlap (e.g. $TIR_2$, $TIR_5$, $TIR_6$ and $TIR_8$ in Figure 1(c) (126)) are more likely to represent credible insertion regions within the HPC genome than sequences that are characterized by large overlaps of their number of reads (e.g. $TIR_1$, $TIR_7$ and $TIR_{10}$ in Figure 1(c) (126)). This is because putative TIRs characterized by a high degree of exact overlap likely represent PCR artifacts from the amplification process. The obtained RTIRs in the randomly chosen subclone (RSC) are then used for comparison (127) with the corresponding comparative transgene insertion regions (CTIRs) in the one or more subclones (SCs), determined analogously to the process set out above. CTIRs in a given SC genome may for instance be compared with the corresponding RTIRs in the RSC in a presence/absence matrix as a binary color code (128).

In such a matrix, one color (e.g. black in (128)) indicates that a CTIR in a respective SC is congruent with a corresponding RTIR in the RSC. Conversely, another color (e.g. white in (128)) indicates that a CTIR in a respective SC is not congruent with a corresponding RTIR in the RSC. In the illustrative presence/absence matrix (128), the congruence of all CTIRs in $SC_2$, $SC_3$ and $SC_5$ with each of the RTIRs in the RSC suggests that $SC_2$, $SC_3$ and $SC_5$ are each identical in genetic makeup to each other as well as to the RTIR with respect to the locations of transgene insertion. In contrast, the presence of white boxes in the columns corresponding to $SC_1$ and $SC_4$ indicate that certain RTIRs in the RSC are absent in these SCs; $SC_1$ and $SC_4$ are not identical to each other or to the RSC with respect to the locations of transgene insertion.

Since each of the SCs and the RSC resulted from independent expansion of the same (putatively monoclonal) MCB, in principle a deviation at any one CTIR in a given SC from the corresponding RTIR in the RSC (i.e. a white box anywhere in the presence/absence matrix (128)), is an indication that the MCB which was originally assumed, based on statistically limiting dilution, to be monoclonal, is actually not monoclonal, but likely contained multiple, genetically distinct cells in the same well Or the sequences coverage is too little and insertion sites are present in the SC but technically missed since not sequence reads where obtained.. Alternatively and original clonal cell lines has genetically changed in a manner that some cell have lost certain insertion sites whereas others have maintained the original transgenes insertion sites which indicates that the MCB is unstable and has become polyclonal. Conversely, congruence between each and every CTIR in each evaluated SC with the corresponding RTIR in the RSC (i.e. black boxes everywhere in the presence/absence matrix (128)) is an indication that the original assumption that the MCB was monoclonal was correct, and that there was indeed only one cell in the initial well following statistically limiting dilution. The presence/absence matrix (128) may therefore be viewed as a qualitative indication of clonality. Clonality between the RSC and the one or more SCs may be additionally quantitatively assessed e.g. by calculating the Dice coefficient for each of the SCs assessed. The Dice coefficient is an expression of the degree of similarity between the RSC and a respective SC based on the degree of congruence between RTIRs in the RSC and corresponding CTIRs in a given SC. In this way, the results obtained from the presence/absence matrix (128) may be additionally represented in a distance matrix (130). The distances of the respective SCs with respect to the RSC may be represented as dots along a scale from 0 to 1 in a two-dimensional distance matrix (130), with 0 indicating complete clonal identity in respect of all assessed TIRs, and 1 representing complete clonal dissimilarity in respect of all assessed TIRs. Dots that superimpose on one another form a so-called "cluster", which is a group of respective SC genomes exhibiting the same distance from, and therefore genetic congruence with, the RSC genome. This is for example the case in (130) for $SC_2$, $SC_3$ and $SC_5$. The one or more respective SC genomes are considered to belong to the same cluster compared to the RSC genome if the distance between them as calculated based on the Dice coefficient is 0 (i.e. as long as the data points superimpose). A Dice coefficient of "0" between two respective clones means that the two clones in question share genomic identity, i.e. they have congruent transgene insertion regions. If the RSC and each of the one or more SCs are grouped into the same cluster, the MCB for which clonality has to be determined is considered to be monoclonal.

The above means that if all loci in a presence/absence matrix, including those for the RSC and all SCs, are the same color, then one may conclude monoclonality. If the results from the presence/absence matrix are expressed as numerically by Dice coefficient, then the above means that monoclonality may be concluded if all data points corresponding to the SCs superimpose at a single point, and a single point corresponds to dice coefficient equals 0.

The degree of certainty increases with the number of TIR compared and found to be congruent. Ultimately if the entire genomes of RSC and SC are completely sequenced this would result in 100% certainty of a given SC being identical to an RSC. The larger the number of SCs analyzed the more likely it is to determine whether an MCB is monoclonal. In an ideal case several hundred or even thousand of clones are analyzed in such a way.

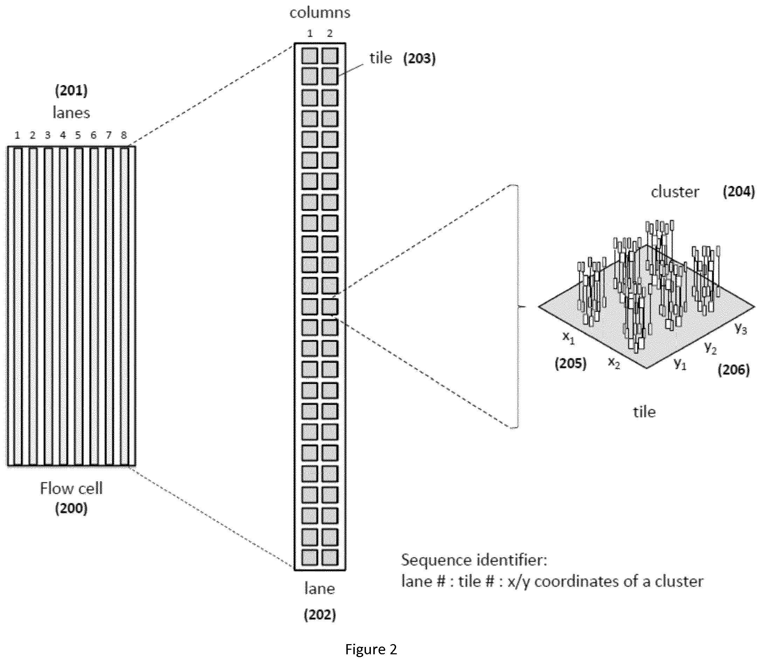

Figure 2 illustrates the schematic design of an illustrative flow cell (200) on which the DNA molecules of a given DNA library are immobilized, "bridge" amplified to generate clusters and sequenced to obtain million of copies of a respective DNA molecule. A flow cell is divided into 8 lanes (201) and each lane (202) comprises 50 tiles (203) to which the DNA molecules are bound via adapter hybridization. The DNA molecules are then amplified to generate thousands of copies of a single DNA molecule ("clusters" (204)). To each of these clusters "x" and "y" coordinates can be assigned during sequencing to precisely identify the exact location of a given cluster (204) or DNA molecule within the tile (203) of the flow cell (200). During next-generation sequencing (NGS) raw data is generated. This raw data is then converted into FastQ files. FastQ files store the biological sequence of the DNA molecule and its corresponding quality scores in a text-based format. The FastQ files further contain a sequence identifier comprising information about the unique instrument name used for sequencing, the lane of the flow cell, the tile number within the flow cell and the "x" (205) and "y" (206) coordinates of a DNA cluster (204) corresponding to a given DNA molecule within the tile (203). The sequence identifier further comprises information about the member of a given paired-end sequencing read (i.e. it identifies a given sequence either as "read 1" or as "read 2"). Based on this sequence identifier it is therefore possible to exactly identify the position, or address, of a DNA cluster or DNA molecule within the flow cell (200). More importantly, it is possible to identify corresponding read pairs (i.e. read 1 and read 2) generated in the paired-end sequencing step based on information of the sequence identifier stored in FastQ files generated for sequenced DNA molecule.

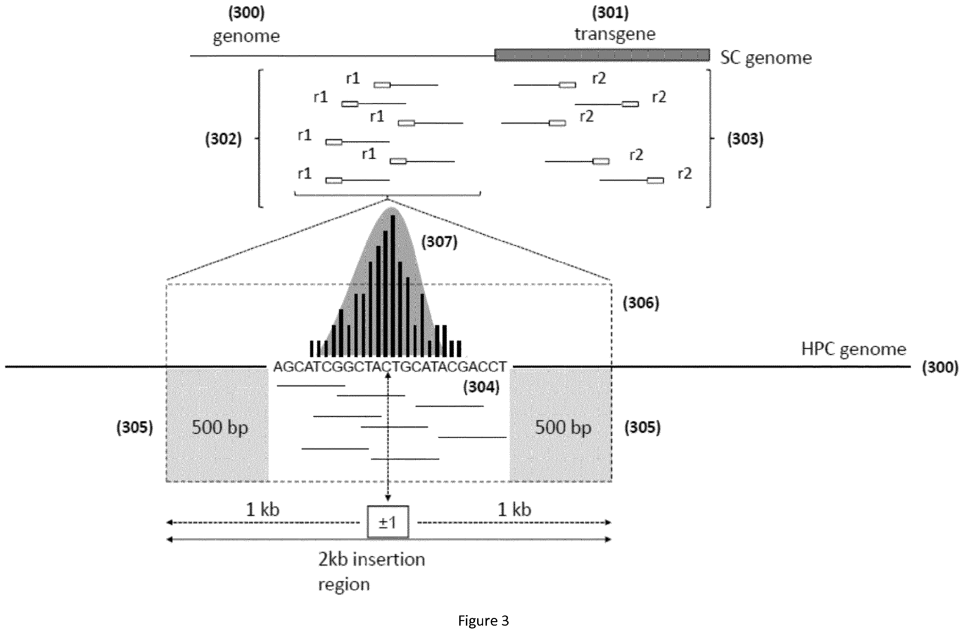

Figure 3 illustrates the identification of transgene insertion regions (TIRs) by alignment of class 2 reads (302) to the HPC genome (300) and class 3 reads (303) to the transgene (301). It would similarly be possible to depict class 1 and class 4 reads on the other side of transgene (301) in Figure 3, but analogous to the description for Figure 1 above, these have been omitted for reasons of conciseness. The aligned reads span a region of approximately 1000 nucleotides (1 kb) (304) within the HPC genome (300), which defines the putative region of transgene insertion. To increase statistical robustness and render the prediction of TIR location more credible, the putative regions of transgene insertion are extended to a 2 kb window (306) starting from the center of the 1 kb region (304) and extending on both sides by a region of 500 bp (grey boxes (305)). The center of the 1 kb insertion region is defined as the nucleotide in the HPC genome with the highest sequence coverage (see Gaussian distribution (307)). Considering a 2 kb insertion region of the transgene makes the prediction of TIRs in the HPC genome much more credible because it allows for a more reliable comparison of TIRs between different subclones even in the event that small variations in the sequence alignment of the one or more subclones occur. Furthermore, the alignment of class 2 (302) and class 3 (303) sequences to the HPC genome (300) does not represent the exact position of the transgene insertion region in the HPC genome because the sequences corresponding to read 1 or read 2 of a paired-end sequencing pair usually span only 200 to 500 bp. The template to be sequenced, however, can be 800 bp or more in length (determined by the average insert size of the DNA library). This difference can potentially lead to a gap of 300 to 600 bp in which the real transgene insertion site might be located. Thus, expanding the predicted transgene insertion region by an additional region of 500 bp on each site of the 1 kb region increases the credibility of the predicted TIRs because this expansion takes into account possible gaps in the sequences in which the actual transgene insertion sites might be located.

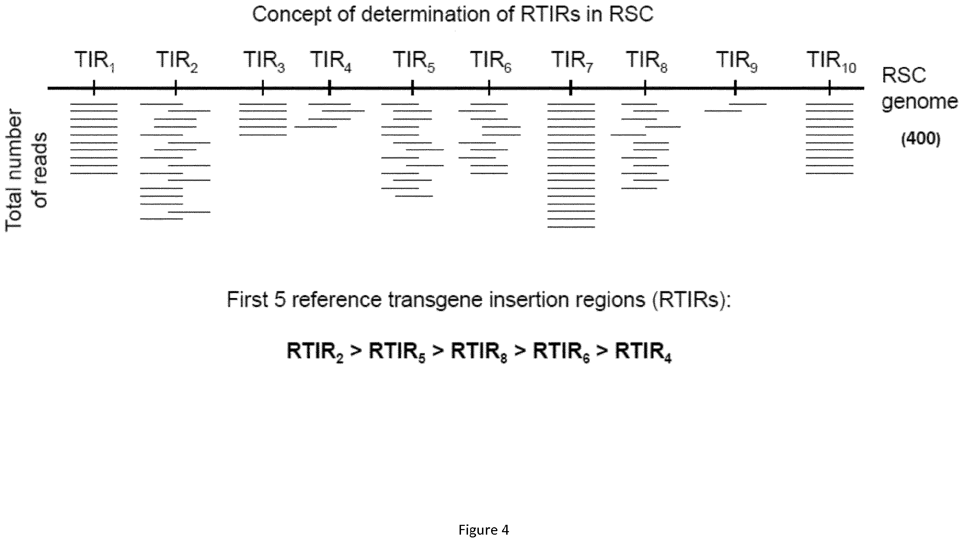

Figure 4 illustrates the concept of determination of the reference transgene insertion regions (RTIRs) in the RSC genome (400). The identified TIRs in the RSC are analyzed for their sequence coverage and the number of reads to obtain credible TIRs that can be used as references for the comparison of TIRs in the one or more SCs. The RSC is sequenced with higher sequence coverage to allow a more reliable prediction of transgene insertion regions in the RSC genome (400). In particular, higher sequence coverage avoids the identification of false TIRs in the RSC genome due to PCR artifacts. RTIRs are selected based on the total number of sequence reads and a low overlap of sequence reads. The reason for this is that PCR artifacts tend to generate multiple copies, which are exactly the same sequence, whereas many partially overlapping sequences converging to a single location can have originated only from random fragmentation of the host genome during library preparation. The former should be excluded as not truly predictive of the locations of the (R)TIRs, while the latter should be included.

To illustrate this, Figure 4 shows 10 different transgene insertion regions ($TIR_1$ to $TIR_{10}$) located in the RSC genome. Although $TIR_7$ has the highest sequence coverage of all ten TIRs (i.e. highest number of overlapping reads) it likely does not represent a credible TIR because the reads at $TIR_7$ are identical. The same applies for $TIR_1$, $TIR_{10}$ and $TIR_3$; while the number of overlapping reads at each of the 3 latter TIRs is lower than the number at $TIR_7$, As explained above, such "pile-up" sequences are more likely due to sequencing "artifacts" that might have been introduced during library preparation or PCR amplification e.g. due to sequence biases, but do not represent reliable transgene insertion regions that are suitable as references for the comparison of the one or more SCs with the RSC. Based on the above requirements, the five best insertion regions, in order of descending reliability, would therefore be $TIR_2$ (e.g. $RTIR_2$) followed by $RTIR_5$, $RTIR_8$, $RTIR_6$ and $RTIR_4$. Each of these five TIRs exhibit partial sequence overlap, with higher numbers of partially overlapping reads at one location denoting higher reliability.

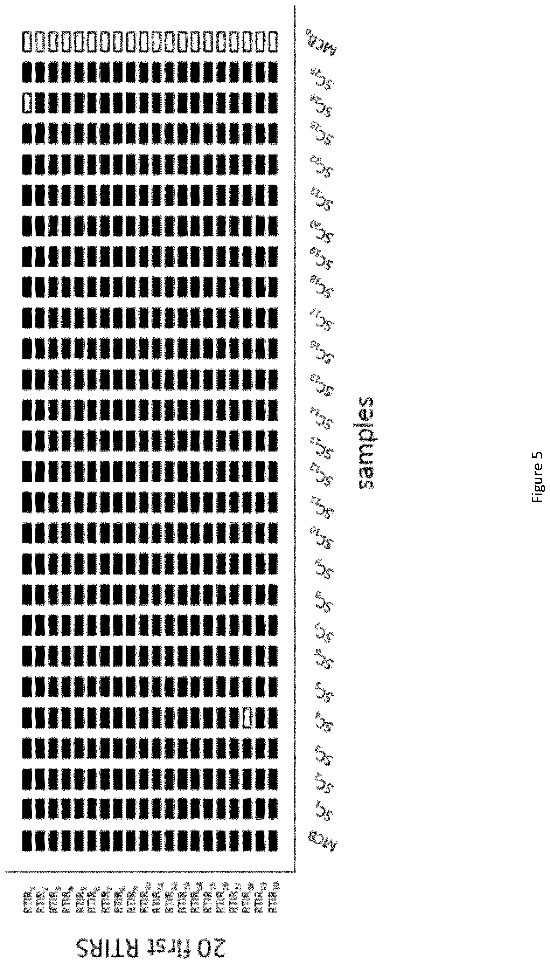

Figure 5 illustrates the results (e.g. of Example 5) obtained by the comparison of the transgene insertion regions (TIRs) between exemplary 25 subclones, the MCB and a divergent $MCB_\Delta$ (the $MCB_\Delta$ resulting from gene insertion different from the MCB), displayed in a presence/absence matrix as described above. The "y" axis of the presence/absence matrix represents the positions of 20 RTIRs (e.g. the 20 RTIRs with the highest sequence coverage, as described above) in the genome of subclone #25 ($SC_{25}$). In the exemplary figure, $SC_{25}$ is sequenced on 3 lanes of the flow cell to obtain a higher degree of sequence coverage as compared to other relevant libraries (i.e. libraries of the MCB, $MCB_\Delta$ and $SC_1$ to $SC_{24}$ which are each sequenced on 1 lane of the flow cell (see Table 1; column 2). This higher sequence coverage and corresponding higher robustness in the results obtained for $SC_{25}$ therefore justifies assignment of $SC_{25}$ as the RSC. The positions of the 20 RTIRs are thus determined based on the higher sequence coverage in the $SC_{25}$ library as compared to the sequence coverage of the other sequenced libraries.

The "x" axis represents each of the 25 tested subclones, the MCB and the $MCB_\Delta$. As indicated by the binary color code, none of the 20 RTIRs identified in $SC_{25}$ is present in the $MCB_\Delta$, whereas the MCB and the majority of the tested subclones (i.e. $SC_1$ to $SC_3$ and $SC_5$ to $SC_{23}$) share the 20 RTIRs with $SC_{25}$, the RSC (see "white" bands in the very right lane representing $MCB_\Delta$). This result confirms that the $MCB_\Delta$ is the result of a random transfection different from that leading to the MCB, resulting in the insertion of the transgene at different positions in the HPC genome. Furthermore, the exemplary data show that the MCB and the majority of the 25 subclones share all identified RTIRs (except for $SC_4$ and $SC_{24}$), indicating that these SCs originated from a single monoclonal cell (MCB). $SC_4$ and $SC_{24}$, however, appear to lack one RTIR in their respective genome. $SC_4$ apparently lacks $RTIR_{18}$, whereas $SC_{24}$ apparently lacks $RTIR_1$.

In cases such as these, in which almost all RTIRs are present in a given SC, there is a high probability that the SC in question is in fact identical in its TIRs to the RSC, and that the deviation at a single TIR in the SC from its corresponding RTIR in the RSC is due to a sequencing artifact.

To exclude this possibility, and thus determine definitively whether a given SC is identical to the corresponding RSC, it can therefore be useful to further analyze the aberrant TIR in question. This may for example proceed by Sanger sequencing. If such further sequencing analysis shows that the observed deviation of the TIR in the SC from the corresponding RTIR in the RSC is an aberration, and that the SC is in fact identical to the RSC at this position, one may then reasonably conclude that the apparent absence of the TIR in the SC might result from low sequence coverage, but not from a real absence, of the respective TIRs in the respective SC. In such a case, the presence of e.g. $RTIR_{18}$ in $SC_4$ and $RTIR_1$ in $SC_{24}$ may be confirmed, indicating that all tested subclones share the same (R)TIRs with the MCB for which clonality is to be determined. In such a case, one may conclude that the initial assumption, based on limiting dilution, that the MCB is monoclonal was correct, and that the MCB is in fact monoclonal. Monoclonality of a given MCB can be concluded based on a presence/absence matrix if the TIR profile in all SCs deriving from the MCB is identical to the RTIR profile in the RSC.

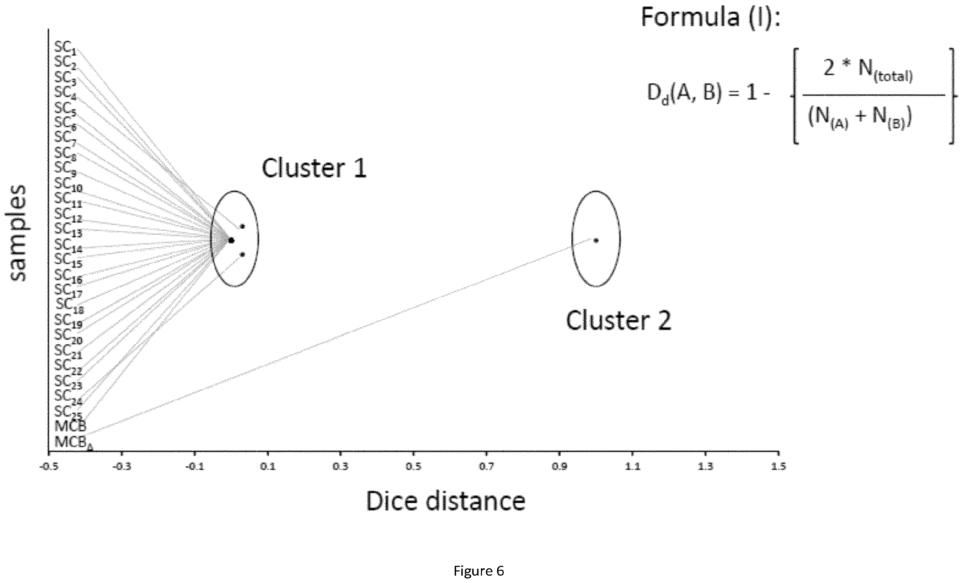

Figure 6 shows a distance matrix spatially representing the congruity between RTIRs/TIRs in the MCB, each of $SC_1$ to $SC_{25}$ and the divergent $MCB_\Delta$. The depicted distances are based on calculation of the respective Dice coefficients according to formula (I), showed herein and in Figure 6. As can be seen from the calculated distance matrix all subclones, except for $SC_4$ and $SC_{24}$, are grouped into the same cluster with respect to the MCB for which clonality is to be determined. $SC_4$ and $SC_{24}$ slightly diverge from this cluster due to the apparent lack of $RTIR_1$ in $SC_{24}$ and $RTIR_{18}$ in $SC_4$. However, as explained above for Figure 5, further analysis of divergent TIRs can be performed by other methods (e.g. Sanger sequencing) to determine whether the apparently absent TIRs are in fact present. In this case, the presence/absence matrix (Figure 5) and the corresponding distance matrix (Figure 6) may be corrected accordingly. Monoclonality of a given MCB can be concluded based on a distance matrix if the calculated distance according to formula (I) is "0" for the subclones tested. As expected, genetically distinct $MCB_\Delta$ has a calculated distance of "1" indicating that this clone was derived from an independently transfected cell.

**DEFINITIONS**

Clonality

As used herein, the term "clonality" describes the genetic constitution of a cell in question, in particular with regard to the similarity or dissimilarity to a reference genetic constitution. For instance, a "clone" refers to a group of genetically identical cells that share a common progenitor, i.e. they are derived from a single cell and therefore have identical genomes. The term "monoclonal" as used herein describes a group of genetically identical cells, which are derived from a single genetically identical progenitor cell. Monoclonal cells are defined as a group of cells produced from a single progenitor cell by repeated cellular replication and thus can be set to form a single "clone" with similar gene expression profiles and proliferation characteristics. In particular, the term "monoclonal" as used herein refers to a group of cells or clones that share identical genomic locations of transgene insertion. The term "polyclonal" as used herein refers to a group of cells derived from more than one progenitor cell, which are genetically distinct from one another. Polyclonal cells comprise a mixture of more than one cell with different genetic origins having different gene expression profiles and/or proliferation characteristics. In particular, the term "polyclonal" as used herein refers to a number of cells or clones that differ in their genomic locations of transgene insertion.

Master Cell Bank (MCB)

The term "Master Cell Bank" (MCB) as used herein refers to an aliquot of a pool of cells that has been prepared from the selected cell clone under defined conditions, dispensed into multiple containers, and stored under defined conditions. The MCB is derived from a transfected host progenitor cell that incorporated a recombinant nucleic acid sequence (or transgene), usually comprising a gene of interest, in its genome (i.e. the recombinant nucleic acid sequence is then comprised in the MCB). The positively transfected cells are then cultured under selection conditions to obtain a pool of polyclonal cells that incorporated the transgene of interest in the genome, albeit at genomic locations which differ from cell to cell. This pool of cells is then tested for the best candidates for transgene expression by a series of limiting dilutions (i.e. dilution to result in less than one cell per intended aliquot volume) combined with analyses of protein expression and proliferation profiles. Best candidates are then further diluted to statistically yield one single cell from which the MCB then originates. These MCB candidates (pre-MCBs) are then further tested for protein expression and various specific characteristics including cell morphology, protein expression levels, stability of expression, proliferation rates, and the product quality.. Furthermore, the MCB should be tested for endogenous agents, e.g. retroviral, fungal or mycoplasma contamination. The established MCB with preserved characteristics thus represents a "cell reserve" which is stored under defined conditions and which can be used as a production cell line for e.g. recombinant protein expression.

Host progenitor cell

The term "host progenitor cell" (HPC) as used herein refers to a cell, which serves as a host for incorporation, e.g. genomic incorporation, of a recombinant nucleic acid sequence, also named transgene. The recombinant nucleic acid sequence usually comprises a Gene Of Interest (GOI) encoding e.g. a therapeutically relevant protein. In some instances, the recombinant nucleic acid sequence may also comprise a therapeutic DNA or RNA, e.g. DNA-aptamers or siRNA. The recombinant nucleic acid sequence may be introduced and stably integrated into the HPC genome by known transfection methods. HPCs are commonly of mammalian origin and include e.g. Chinese hamster ovary (CHO) cells, mouse myeloma (NS0, SP2/0), baby hamster kidney (BHK), human embryo kidney (HEK-293) and human retinal cells, but are not limited to these.

Stable gene integration

The term "stable gene integration" or grammatically related terms such as "stably integrated", etc. refers to the incorporation of a given transgene comprising a GOI into the HPC genome, so that the transgene comprising the GOI is maintained in the host cell genome during cell proliferation cycles and is replicated together with the host genome and appears in progeny cells. A cell line having undergone stable gene integration has, therefore, incorporated a transgene comprising a GOI in its genome and its daughter cells will also contain the transgene comprising the GOI in each replicated daughter cell.

When developing a stable transfection, the use of selectable markers, for example also comprised in the transgene, to distinguish transient from stable transfection is advantageous. Co-expression of the marker with a given GOI helps to identify and select for cells that have the given recombinant nucleic acid sequence integrated into their genomes, while also selecting against the transiently transfected cells, i.e. cells that have not incorporated the given recombinant nucleic acid sequence with the marker and the GOI into their genomes. For example, a common selection method is the transfection of a transgene encoding a GOI and a gene conferring antibiotic resistance (e.g. the neomycin resistance gene, neo). The transiently transfected cells are then treated with the appropriate antibiotic for selection (e.g. geneticin or G418 for neo-transfected cells). Only those cells that have stably integrated the recombinant nucleic acid sequence comprising the GOI and the gene conferring antibiotic resistance will survive in long-term cultures, allowing for selection and expansion of the desired cells that have stably integrated the transgene.

Retroviral transfection

Retroviruses are single-stranded RNA viruses that stably integrate into the host cell's genome via a double-stranded DNA intermediate. Retroviral vector systems such as the murine leukemia virus derived vectors can therefore be used as efficient tools to stably integrate a recombinant nucleic acid sequence into the host progenitor cell (HPC) genome, and this is the meaning of "retroviral transfection" as used herein. A retroviral vector may comprise proviral sequences that can accommodate the recombinant nucleic acid sequence to allow the latter's incorporation into the HPC genome. The vector may also comprise viral and cellular gene promoters, such as the strong CMV promoter, to enhance expression of the transgene, including the GOI, in the host progenitor cell. Virus-mediated transfection results in the random or quasi-random insertion e.g. preferred insertion in transcriptionally active sites in the HPC genome of the recombinant nucleic acid sequence at multiple sites in the HPC genome.

Recombinant nucleic acid sequence

The term "recombinant nucleic acid sequence" as used herein refers to a genetically engineered nucleic acid molecule, for example a genetically engineered DNA molecule, which is generated by laboratory methods (e.g. molecular cloning). It is also named a transgene. The recombinant nucleic acid sequence usually comprises a Gene Of Interest (GOI) intended for ultimate expression in the MCB. It may additionally comprise additional nucleic acid sequences helpful or required for identification of stably transfected cells (e.g. antibiotic resistance markers and the like) and/or for facilitating expression of the GOI in the MCB. The recombinant nucleic acid sequence may comprise genetic material (e.g. DNA fragments) from different biological sources (e.g. cells or organisms), thereby creating a recombinant sequence that would not otherwise exist in nature and that can be introduced into the genome of a host progenitor cell. The recombinant nucleic acid sequence may alternatively comprise genetic material (e.g. DNA fragments) from a single biological source (e.g. a single cell or organism), in the same or similar form in which this genetic material exists in the biological source, but manipulated and/or isolated by recombinant laboratory techniques known to the skilled person. The recombinant nucleic acid sequence comprises the GOI intended for ultimate expression in the MCB. In the event that no further sequences other than the GOI are comprised in the recombinant nucleic acid sequence, then the terms "recombinant nucleic acid sequence" and "GOI" become identical; in this case the recombinant nucleic acid sequence, or transgene, consists of the GOI. The present disclosure envisions all above variants of the term "recombinant nucleic acid sequence".

Gene of Interest

The term "Gene Of Interest", or "GOI", as used herein refers to a nucleic acid sequence, e.g. a DNA sequence, encoding at least part of a recombinant protein. The GOI is comprised in the recombinant nucleic acid sequence. The GOI encoding the recombinant protein may be taken directly from the genome of an organism or a cell. Alternatively the GOI may be derived from or be identical to the open reading frame resulting from the splicing of multiple genomic exons into a single contiguous nucleic acid sequence encoding the protein of interest, i.e. the GOI may be equivalent to the DNA complementary to (i.e. cDNA) the mRNA encoding the recombinant protein. The GOI may be isolated in complete form from an appropriate biological source, or may be chemically synthesized. The GOI may additionally comprise post-transcriptional modification, e.g. modified nucleosides and/or modified nucleotides. The GOI as part of the transgene can then be introduced into the genome of the host progenitor cell. The GOI can be alone in the transgene, in which case the transgene and the GOI are coextensive).

Recombinant protein and recombinant protein expression

The term "recombinant protein expression" as used herein refers to the expression of a protein in a host cell which is encoded by a given recombinant nucleic acid sequence. In most cases, the protein will be expressed from the GOI comprised in the transgene, i.e. the recombinant protein will be expressed by the GOI. However, it is not excluded that the recombinant protein may be expressed from a combination of the GOI and other sequences within the transgene.
As used herein, the term "recombinant protein" refers to a protein, which is expressed from the recombinant nucleic acid sequence. In many cases, the recombinant protein will be a protein of therapeutic value, and the recombinant nucleic acid sequence will have been stably integrated into the genome of a host progenitor cell to result in an MCB to be used in the production of this protein. As explained above, the protein may be considered "recombinant" by virtue of its being encoded by and/or expressed from a nucleic acid sequence which results from combining, e.g. using known laboratory methodology for the controlled manipulation and/or isolation of genetic sequences *in vitro*, multiple nucleic acid sequences from different biological sources or organisms in a form which does not otherwise exist in nature. The protein may alternatively be considered "recombinant" by virtue of its being encoded by and/or expressed from a recombinant nucleic acid sequence which, although it already exists in nature, was manipulated and/or isolated using known laboratory methodology for the controlled manipulation and/or isolation of genetic sequences *in vitro.* The recombinant protein may be encoded by and/or expressed from the recombinant nucleic acid sequence, including the GOI comprised in the recombinant nucleic acid sequence together with other sequences comprised in the recombinant nucleic acid sequence. Alternatively the recombinant protein may be encoded by and/or expressed from exclusively the GOI comprised in the recombinant nucleic acid sequence, despite the presence of sequences other than the GOI in the recombinant nucleic acid sequence. Alternatively, the recombinant protein may be encoded by and/or expressed from exclusively the GOI comprised in the recombinant nucleic acid sequence when the recombinant nucleic acid sequence comprises no sequences other than the GOI. In this latter case, one may refer to the recombinant protein being encoded by and/or expressed from either the "transgene" or the "GOI", these two entities being identical in the absence of other non-GOI in the recombinant nucleic acid sequence.

Promoter

As used herein, the term "promoter" refers to a sequence-specific site in DNA, which is recognized by transcription factors and RNA polymerase to initiate transcription of mRNA.

Virion

The term "virion" as used herein refers to a complete viral particle consisting of RNA or DNA which is surrounded by a protein envelope and which constitutes the infective form of a virus.

Sequence alignment (mapping)

As used herein, the term "sequence alignment" or "sequence mapping" refers to a way of arranging the sequences of DNA or RNA, relative to one another so as to identify regions of similarity. Such sequences may be a consequence of functional, structural, or evolutionary relationships between the sequences. In the context of the present invention, alignment may in particular be used to elucidate the origin of cells and the nucleic acid comprised within them, for example whether such cells result from a common cellular progenitor or different cellular progenitors. Aligned sequences of nucleotides are typically represented as rows within a matrix. The terms "alignment" and "mapping" as used herein have the same meaning, and are thus interchangeable with each other. Well-known algorithms for sequence alignments are for instance Needleman-Wunsch algorithm, Smith-Waterman algorithm or Waterman-Eggert algorithm or Burrows-Wheeler transform. Well-known tools for sequence alignments are for instance BLAST, BLAT, WMBOSS, Clustal, BWA, Bowtie.

Subclone

The term "subclone" as used herein refers to a pool of cells which has been isolated from a master cell bank (MCB) as a single cell for example as a result of limiting dilution (i.e. dilution to result in no more than one cell per intended aliquot volume) and subsequently expanded to a pool of cells. All the cells of the subclone share identical genomic organization. In the present invention, subclones are expanded from a given MCB culture comprising of a pool of cells that are assumed to be monoclonal. The MCB is therefore split into different cell aliquots wherein each cell aliquot represents a particular subclone. After expansion, the subclones can then be used for e.g. protein expression, clonality analysis and the like. Subclones that originate from a monoclonal MCB (a pool of cells that originate from one single cell) share identical genomic characteristics and each subclone is therefore considered to be monoclonal in relation to any other subclone originating from the same, i.e. common MCB. Subclones that originate from a polyclonal MCB (a pool of cells that originate from at least two different cells) will have different genomic characteristics and protein expression profiles and will therefore be considered as polyclonal in relation to each other.

Reference subclone (RSC)

The term "reference subclone" (RSC) as used herein refers to a subclone which has been randomly chosen from a group of subclones expanded from an MCB, the clonality of which is to be determined. The RSC is sequenced with a higher sequence coverage compared to the remaining subclones to obtain a higher average number of reads representing a given nucleotide in the reference sequence. The sequencing data of the RSC is used to identify the one or more reference transgene insertion regions (RTIRs).

Reference transgene insertion regions (RTIRs)

The term "reference transgene insertion region" (RTIR) as used herein refers to a transgene insertion region identified in the genome of the reference subclone (RSC). Transgene insertion regions are assigned as RTIRs if they have a high sequence coverage and a low overlap of read number of readsnumber of reads compared to other transgene insertion regions, as explained in detail herein. RTIRs in the RSC are compared with corresponding transgene insertion regions in one or more, best multiple, subclones originating from the same MCB as the RSC in order to determine clonality of the MCB.

Comparative transgene insertion regions (CTIR)

The term "comparative transgene insertion region" (CTIR) as used herein refers to a transgene insertion region in one or more subclones originating from the same MCB as the RSC, said transgene insertion region being compared to an RTIR in the RSC at the corresponding genomic position.

DNA sequencing library

The term "DNA sequencing library" as used herein refers to a sample of genomic DNA fragments purified from a particular biological source (e.g. an MCB, RSC, SC or $MCB_A$) representing the entire genome of said biological source. In a DNA sequencing library the genomic DNA fragments may be 3'- and 5'-ligated to primers and adapter sequences for further analysis of the genomic DNA fragments (e.g. sequencing analysis).

For example, the preparation of a DNA library for sequencing may start with the fragmentation of the DNA sample, which was purified from a particular biological source. The fragmentation defines the molecule entry points for the sequencing reads. In a next step, DNA ends may be enzymatically repaired and adenine (A) may be added to the 3' ends of the DNA fragments. The (A)-tailed DNA fragments may then be amplified as templates to ligate double-strand, partially complementary adapters to the DNA fragments. The DNA library may then be size-selected and amplified to improve the quality of sequence reads. The amplification reaction introduces specific PCR primers to the adapter sequences that are required for sequencing on the flow cell.

Single cell Sequencing

Single cell DNA genome sequencing involves the isolation of a single cell and the subsequent whole-genome-amplification, and then the DNA sequencing using a next-generation sequencer. Single cell sequencing examines the sequence information from individual cells.

Next-generation sequencing

As the skilled person understands, nucleic acid sequencing is a method for determining the exact order of nucleotides in a given nucleic acid molecule. The term "next generation sequencing" (NGS) as used herein refers to any sequencing platform or sequencing technology, which allows parallel simultaneous sequencing of many nucleic acids. This allows many, for example millions, of fragments of DNA from a single sample to be sequenced in parallel. NGS technology therefore enables sequencing up to 1 nucleotide resolution and allows an entire genome to be rapidly sequenced, e.g. in a matter of hours. "NGS methodology" and "NGS technology" as used herein comprises template preparation, sequencing and imaging, and data analysis.

For instance, the Illumina/Solexa® approach achieves DNA amplification by attaching single-stranded DNA fragments to a solid surface known as a single molecule array, or flow cell, and conducting the solid phase "bridge" amplification of single molecule templates. In this process, one end of a single DNA molecule is attached to a solid surface using an adapter; the molecules subsequently bend over and hybridize to complementary adapters thereby creating a "bridge" which forms the template for synthesis of the complementary strand. After amplification, the flow cell may contain more than 40 million clusters, wherein each cluster comprises as many as 1000 clonal (identical) copies of a single template molecule. The templates are sequenced in a parallel fashion using the DNA sequencing-by-synthesis approach (see definition below) that employs reversible terminators with removable fluorescence moieties and special DNA polymerases that can incorporate these terminators into growing oligonucleotide chains. The terminators are labeled with four different colors to distinguish among the different bases at a given sequence position and the template sequence of each cluster is then determined by the color readout of each fluorophore upon each successive nucleotide addition. This readout occurs through a cycle of washing and flooding the fragments with known nucleotides in a sequential order.

Once sequencing is complete, raw sequence data must undergo several analysis steps. A generalized data analysis pipeline for NGS data includes pre-processing the data to remove adapter sequences and low-quality reads, mapping of the data to a reference genome or *de novo* alignment of the sequence reads, and analysis of the compiled sequences. Analysis of the sequences can include a wide variety of bioinformatic assessments, including assessment for genetic variants calling for detection of small nucleotide polymorphisms (SNPs), detection of novel genes, transgene insertion sites, and/or assessment of transcript expression levels.

Flow cell

The term "flow cell" as used herein refers to a multi-lane, typically glass-based substrate in which the nucleic acid clusters are generated by "bridge" amplification and the sequencing step is performed. Each of the lanes is individually addressable, so it is possible to sequence multiple distinct samples per flow cell.

Within each lane of the flow cell, millions of primers act as capture probes for the fragmented DNA libraries. Each lane of the flow cell is capable of yielding millions of distinct nucleic acid clusters each of which comprises a particular DNA fragment to generate a high depth of sequencing data, i.e. distinct DNA fragments are "bridge amplified" and sequenced a million times giving rise to reliable sequencing results.

Sequencing by synthesis

As used herein, the term "sequencing-by-synthesis" denotes a real-time method in which the fluorescence signal is directly detected after incorporation of the fluorescently labeled nucleotide and before incorporation of the next nucleotide. Specifically, the method uses four nucleotides, each fluorescently labeled with a different colored fluorophore, to sequence the nucleic acid clusters on the flow cell in parallel. During each sequencing cycle, a single labeled, reversible terminator, deoxynucleotide triphosphate (dNTP) is added to the nucleic acid chain. The nucleotide label serves as a terminator for polymerization, so after each dNTP incorporation, the fluorescent dye is imaged to identify the base and then the 3' terminator block is enzymatically cleaved to allow incorporation of the next nucleotide. Since all four reversible terminator-bound dNTPs (A, C, T and G) are present as single, separate molecules, natural competition minimizes incorporation biases. Base calls are made directly from signal intensity measurements during each cycle. The end result is a base-by-base sequencing which enables reliable base calling and elimination of sequence-context specific errors.

Base calling is the process of assigning a nucleotide to a specific fluorophore read out.

Basecall files (.bcl) are binary file containing base call and quality for each tile in each cycle.

Paired-end sequencing

As used herein, the term "paired-end sequencing" refers to a process in which a single fragment is sequenced from both ends, 5' and 3', giving rise to forward (read 1) and reverse (read 2) reads. The sequenced fragments might be separated by a gap of certain bases or might overlap, giving rise to a contiguous longer single-end fragment after merging. Using paired-end reads improves the accuracy of reads mapping onto the reference genome or the transgene.

FastQ files and sequence identifier

The FastQ format is a text-based format for storing the biological sequence (e.g. a nucleotide sequence or peptide sequence) using a single letter code and its corresponding quality scores (FastQ was developed by Wellcome Trust Sanger Institute). Both the sequence letter and quality score are each encoded with a single ASCII (American Standard Code for Information Interchange) character. A FastQ file consists of 4 lines per sequence. Line 1 begins with a "@" character and is followed by a sequence identifier and an optional description. Line 2 represents the raw sequence letters. Line 3 begins with a "+" character and is optionally followed by the same sequence identifier (and any description) again. Line 4 encodes the quality values for the sequence and must contain the same number of symbols as letters in the sequence, i.e. for each character representing a particular nucleotide in the nucleic acid sequence a corresponding character representing the quality score for the particular nucleotide exists. The "sequence identifier" for each sequencing read contains information about the unique instrument name used for sequencing, the flow cell lane, the tile number within the flow cell lane, the "x" coordinate of the cluster within the tile, the "y" coordinate of the cluster within the tile, the index number for a multiplexed sample and the member of a pair (/1 or /2 for paired end reads only). Based on this sequence identifier read 1 sequences can be combined with corresponding read 2 sequences of corresponding sequence pairs generated during paired-end sequencing.

Number of reads

The term "number of reads" as used herein refers to the number of times a respective nucleic acid molecule is amplified during the NGS process. The "number of reads" is a direct measure of the abundance of a respective nucleic acid molecule in a given nucleic acid library.

Sequence coverage

The term "sequence coverage" as used herein refers to the number of reads that cover each genome base pair.

"High" or "higher" sequence coverage

The term "high" or "higher" sequence coverage as used herein describes a nucleic acid library in which the average number of reads representing a given nucleotide is greater than the average number of reads representing a corresponding nucleotide in another nucleic acid library. This is essentially an expression of the read-redundancy with which any given genomic position is sequenced, with higher levels of read-redundancy correlating to higher sequence coverage (also sometimes termed "deeper sequencing"). For example, "higher" sequence coverage of a given nucleic acid library can be achieved by sequencing a given nucleic acid library on multiple, i.e. redundant, lanes of the flow cell. Higher degrees of sequence coverage allow for a more robust and therefore more statistically reliable analysis of the sequencing data than possible when employing lower degrees of sequence coverage.

PHRED quality score

As used herein, the term "PHRED quality score" (Q) refers to a property which is logarithmically related to the base-calling error probabilities (Ewing B and Green P (1998). Base-calling of automated sequencer traces using phred. II. Error probabilities. Genome Res. 8: 186-194.). PHRED quality scores are calculated based on the formula $Q = - 10 \log_{10}P$, wherein P is defined as the base call error probability. For example, if PHRED assigns a Q score of 30 (Q30) to a base, this is equivalent to the probability of an incorrect base call 1 in 1000 times. This means that the base accuracy (i.e. the probability of a correct base call) is 99.9%. A lower base call accuracy of 99% (Q20) will have an incorrect base call probability of 1 in 100, meaning that every 100 bp in a given sequencing read will likely contain an error. When sequencing quality reaches a PHRED of Q30, virtually all reads will be perfect, having no errors or ambiguities.

Phasing and prephasing

The term "phasing" as used herein refers to the incorporation of a nucleotide into a small portion of DNA strands in a given cluster one position behind (-1 nt) of the correct nucleotide of the genomic template in a given sequencing cycle. The term "pre-phasing" as used herein refers to the incorporation of a nucleotide into a small portion of DNA strands within a given cluster one position ahead (+1) of the correct nucleotide of the genomic template in a given sequencing cycle. For instance, a small portion of strands may become out of phase with the current cycle, either falling a base behind (phasing) or jumping a base ahead (pre-phasing) with respect to the correct nucleotide at a particular position on the template. Phasing and pre-phasing are caused by incomplete removal of the 3' terminators and fluorophores, sequences in the cluster missing an incorporation cycle, as well as by the incorporation of nucleotides without effective 3' terminators.

Contig

The term "contig" as used herein refers to a contiguous sequence of DNA created by assembling overlapping sequenced fragments of a nucleic acid sequence, for example originating from a chromosome.

Scaffold

The term "scaffold" as used herein refers a series of contigs that are in the right order but not necessarily connected in one continuous stretch of sequence.

Cluster

The term "cluster" can have different meanings depending on the context in which it is used. If the term "cluster" is used in the context of describing the genetic relatedness or genetic non-relatedness of two or more sequences to one another, the term as used herein refers to a group of independent subclones having identical transgene insertion sites. Subclones which are grouped into the same cluster are considered to be monoclonal. If the term "cluster" is used in the context of the physical location of nucleic acid sequences, the term as used herein refers to a the group of identical copies of a respective "bridge-amplified" nucleic acid molecule on a flow cell during the sequencing process, e.g. the NGS process, as described herein.

Correspondence

The term "correspondence" as used herein refers to the relationship between two or more subclones determined by the presence or absence of a respective transgene insertion region in each of said two or more subclones. Two or more subclones "correspond" to one another if their transgene insertion regions are congruent, i.e. identical, between the two or more subclones, or between a given subclone or group of subclones and an MCB, the clonality of which is to be assessed. Thus, the term "correspondence" may have the same meaning as the terms "agreement", "congruity" and "identity" and should therefore be understood as interchangeable with each other.

Boundaries:

The boundaries are the host cell genome position where the transgene is inserted. They correspond to the "sides" of the transgene or in other terms the insertion sites.

**DETAILED DESCRIPTION**

As mentioned above, one aspect of the disclosure relates to a method of determining the clonality of a master cell bank (MCB), which is generated by the random insertion of a transgene of known sequence into a host progenitor cell (HPC) genome of known sequence.

MCBs and WCBs serve as expression systems for large-scale therapeutic protein production. An essential requirement for an expression system to be considered an MCB for the production of therapeutic protein is high quality protein expression in the host progenitor cell in quantities suitable for industrial scale production. Cultivated mammalian cell lines have become increasingly important in the production of therapeutic protein products. One major advantage of mammalian expression systems compared to e.g. bacterial or yeast systems is the possibility to effect proper protein folding, post-translational modifications, and product assembly, all of which are important requirements for complete biological activity of the protein product.

In the previous decade, bioprocesses based on mammalian cell systems have been applied in the manufacture of vaccines, diagnostic and therapeutic proteins. The most widely used host mammalian cell systems are Chinese hamster ovary (CHO) cells and HEK 293 (human embryonic kidney) cells. These cells can be transfected by numerous transfection methods, including polyethylenimine (PEI), calcium phosphate or retroviral vectors, and are now widely used for production of recombinant proteins both by transient transfection as well as by the formation of stable cell lines.

Further mammalian cell systems which are suitable for large-scale protein production include, but are not limited to, HeLa, HEK293T, U2OS, A549, HT1080, CAD, P19, NIH 3T3 I, L929, N2a, Human embryonic kidney 293 cells, SP2/0, NS0 (see for instance Manual of industrial microbiology and biotechnology, 3rd edition, chapter 12 "Mammalian cel culture for biopharamcutical production", Jinyou Zhang).

Following transfection, cell lines are selected and expanded under selection in serum-free culture conditions. The transfected cells are a pool of polyclonal cells that incorporated the transgene at different positions in their genomes. This heterogeneity is commonly attributable to the random or semi- random insertion of the transgene into the host genome. Next, the heterogeneous pool of cells is screened for the most efficient protein-producing candidate by a process called limiting dilution. In general, the term "limiting dilution" refers to a dilution, which may be performed in a single step or multiple serial steps, to result in one cell per a given intended aliquot volume. For instance, when it is intended to aliquot a solution into individual wells of a multi-well plate, e.g. 96-well plate, limiting dilution of the polyclonal cell culture results in a cell concentration such that, when the culture is aliquoted in a volume corresponding to a single well, each such well contains (statistically) only one cell or less. The respective cells in separate wells are then further expanded under appropriate conditions to obtain candidate clones. These clones, which theoretically derive from one single progenitor cell, are then tested for their proliferation and protein expression profile. The best clones are then used to generate the master cell bank (MCB).

A major drawback of this approach is the assumption that each candidate clone is actually derived from only one single cell per well. As mentioned above, this assumption is based on a statistical calculation governing the dilution factor prior to aliquoting. However, because this calculation is statistical, it is difficult to eliminate the possibility that a promising MCB was only one of multiple cells in a single well, leading to an undesirably heterogeneous (i.e. polyclonal) mixture of cells when the content of this single well is expanded. This could potentially result in less reproducibility and variances in protein quality within a given MCB. In addition, it would be difficult to comply with applicable regulatory requirements for the production of a protein intended for therapy with such a heterogeneous mixture, as regulatory agencies typically require that the MCB used for production of the therapeutic protein be homogeneous, i.e. monoclonal.

Traditionally, testing of the MCB for high quality production of the protein product is achieved by time-, labor- and cost-intensive assessment of MCB characteristics, including e.g. cell morphology, production stability and protein quality, and genotypic characterization. Such assessments required for a determination of monoclonality typically require 6 to 12 months. However, these parameters are merely indications that the MCB in question is likely to be monoclonal, these tests are still not able to conclusively prove that a given MCB is monoclonal. Currently, the main method accepted by the Health Authorities is the double limited dilution cloning.

The present disclosure relates to a novel method to confirm the clonality of a MCB by the analysis of a selection of unique transgene insertion regions (TIRs) in a given HPC genome of known sequence by means of a novel combined approach of subcloning, sequencing, e.g. next generation sequencing (NGS) and bioinformatic analysis. This novel approach not only avoids the time- and labor-intensive endeavors associated with the traditional identity testing of an MCB, but also allows to conclude on the clonality of a MCB in question, and thus of the MCB's reliability for reproducible protein expression quality during the complete production pipeline.

In general, the method involves the identification of one or more transgene insertion regions (TIRs) in the genome of a random, i.e. randomly chosen, subclone (RSC) expanded from a putative MCB. Therefore, prior to the inventive method, the MCB is expanded to one or more subclones (SCs) and the one or more subclones is/are cultured separately under appropriate conditions. After DNA extraction and library preparation, the DNA libraries from the one or more subclones are analyzed by paired-end sequencing. The DNA library of a (randomly chosen) reference subclone (RSC) is sequenced with higher sequence coverage. As explained elsewhere herein, "higher" sequence coverage in the DNA library of a RSC may be achieved by sequencing the DNA library of the RSC on multiple lanes of the flow cell, thereby generating a higher number of sequencing reads in the DNA library of the RSC as compared to the number of sequencing reads in the DNA libraries of the remaining SCs which are sequenced on only one lane of the flow cell. The sequencing data obtained for the RSC is then used to identify the transgene insertion regions (TIRs) in the RSC genome. The identification of TIRs may be achieved by separate alignment of the obtained sequences to the HPC genome and to the known sequence of the transgene. The identified TIRs in the RSC genome are then analyzed for their sequence coverage and number of readsvariations and one or more TIRs with the highest sequence coverage and partially overlapping number of reads are assigned as reference TIRs (RTIRs). These RTIRs are then compared with comparative TIRs (CTIRs) identified in the genome of the one or more SCs that were independently generated from the MCB and sequenced, said CTIRs being generated in an analogous manner as described above for the RTIRs. Based on the correspondence between the RTIRs present in the RSC genome and the CTIRs present in one or more SC genomes, the clonality of the MCB is then determined.

Generally, correspondence of CTIRs originating from multiple subclones with the RTIRs originating from the randomly chosen reference subclone may be taken as an indication that the MCB is in fact monoclonal as long as a sufficient number of SCs and RTIRs are tested. This is because if the MCB were polyclonal, one would expect to see a divergence between RTIRs and one or more CTIRs resulting from the fact that reference and comparative subclones actually originated from different, i.e. polyclonal, MCB cells. On the other hand, when the CTIRs of each of the comparative subclones correspond to the RTIRs in the random (reference) subclone (and therefore the CTIRs from separate comparative subclones are also identical to one another), then this may be taken as an indication that the randomly chosen reference subclone as well as the comparative subclones all originated from the same MCB cell and, thus, that the limiting dilution discussed above was in fact successful in yielding, as expected, a single MCB cell in a single well of the 96-well plate. In this case, the correspondence between RTIRs and CTIRs in multiple comparative subclones may be taken as proof that the MCB is monoclonal, and therefore suitable for further use in the production of the protein encoded by the transgene.

The identification of the TIRs is achieved by paired-end sequencing. This involves sequencing of a given nucleic acid molecule from both ends of the template, thereby generating pairs of read sequences for a given nucleic acid molecule. Paired-end sequencing has the advantage of increasing the sequence coverage of the template and thus the mapping accuracy; the first time starting from the 5' (read1) end and then starting from the 3' end (read2). As discussed in more detail below, paired-end sequencing is useful in identifying the TIRs in the RSC genome and in the one or more SC genomes.

In one embodiment of the present invention a random subclone (RSC) is sequenced with higher sequence coverage compared to the one or more SCs (see above for the meaning of "higher"). The higher sequence coverage is achieved, e.g. by sequencing the RSC library multiple times or on more than one lane of the flow cell. The higher the sequence coverage in the RSC library, the more reliable the analysis of transgene insertion regions (TIRs) in the RSC will be, and the reliability of the identification of insertion regions (RTIRs) will also increase. As it will become evident in greater detail below, the identification of credible RTIRs in the RSC genome is important for the present invention, as these RTIRs serve as the references for comparing the one or more subclones with the RSC. Incorrectly assigned RTIRs in the RSC might lead to an incorrect assessment of MCB clonality because the evaluation of correspondence between the incorrectly assigned RTIRs in the RSC genome and the CTIRs in the one or more SC genomes might also lead to wrong conclusions about the clonality of the subclones in which the RTIRs are to be compared (as CITRs). In particular, if a given TIR in the RSC would be incorrectly assigned as a RTIR due to low sequence coverage in the sequencing library of the RSC, such a putative RTIR might not correspond to any of the one or more CTIRs in a respective SC genome. Consequently, one may incorrectly conclude that the RSC and the respective SC, and hence the MCB as well, is polyclonal. Thus, the correct and reliable assignment of one or more TIRs in the RSC as RTIRs is important for the present invention and reliable identification of RTIRs is achieved by higher sequence coverage and low sequence overlap in the RSC library.

In a further embodiment of the present invention, the master cell bank (MCB) is generated by transfection of a host progenitor cell (HPC) into which the transgene is randomly inserted at a plurality of positions in its genome. The mammalian cell system used as the HPC may for instance be a Chinese hamster ovary (CHO) cell line. CHO cells are often the mammalian cell line of choice for the production of recombinant protein-based therapeutics for several reasons. CHO cells are capable of adapting and growing in suspension culture, which is ideal for large scale cultures in pharmaceutical industry. CHO cells pose fewer risks than other cells, as only few human viruses are able to propagate in them. This reduces the risk of infectious contamination and spread of viruses in the production pipeline (Boeger et al. (2005). Structural basis of eukaryotic gene expression. FEBS Lett; 579:899-903). Furthermore, CHO cells can grow in serum-free, chemically defined media, ensuring reproducibility between separate batches of cell cultures as well as allowing an exact record of culturing conditions for a MCB as required by health authorities. CHO cells also allow post-translational modifications to recombinant proteins which are compatible and bioactive in humans (Kim et al. (2012). CHO cells in biotechnology for production of recombinant proteins: Current state and further potential. Appl Microbiol Biotechnol; 93:917-30). Specifically, glycosylation of glycoproteins produced by CHO cells are more human-like in the absence of immunogenic α-galactose epitopes (Ghaderi et al. (2012). Production platforms for biotherapeutic glycoproteins. Occurrence, impact, and challenges of non-human sialylation. Biotechnol Genet Eng Rev; 28:147-75). Finally, there exist several well established gene amplification systems which make use of the genomic instability of CHO cells to allow for gene amplification which ultimately results in higher yield of recombinant protein. Despite the advantages set out above for using CHO cells as the MCB, other types of mammalian cells as a set out herein above are also suitable for use in the inventive method.

In a further embodiment the random or quasi-random insertion of the transgene is effected using a retroviral vector transfection system. Retroviruses are RNA viruses that replicate via a double-strand (ds) DNA intermediate. Retroviral vectors can be applied to make stably transformed cell lines. Furthermore, retroviral gene expression is driven by strong promoters which can be subverted to control the expression of transgenes, thereby yielding higher expression levels for a protein of interest. Finally, retroviral systems have a broad host range, allowing the transfection of many different cell types. One retroviral system which may advantageously be used in the present invention for transfection of the HPC is the GPEx® system. This method utilizes replication defective retroviral vectors, derived from Moloney Murine Leukemia virus (MLV) pseudotyped with Vesicular Stomatitis Virus glycoprotein (VSV-G), to stably insert single copies of genomes at multiple genomic locations into the dividing HPC.

In another embodiment of the present invention the transgene insertion regions (TIRs) are identified by classification of paired-end sequencing reads into four classes. Class 1 represents read 1 (i.e. forward) sequences that map to, e.g. exclusively to, the transgene sequence. Class 2 represents read 1 (i.e. forward) sequences that map to, e.g. exclusively to, the HPC genome. Class 3 represents read 2 (i.e. backward) sequences that map to, e.g. exclusively to, the transgene sequences and class 4 represents read 2 (i.e. backward) sequences that map to, e.g. exclusively to, the HPC genome. Said read 1 and read 2 sequences represent respective forward and backward reads corresponding to the 5' and 3' ends of a given nucleic acid molecule within a nucleic acid cluster generated in paired-end sequencing of a nucleic acid library. Sequence reads that map to both references, i.e. the transgene sequence and the HPC genome, are removed from the analysis pipeline because such sequences cannot be properly aligned to the separate reference genomes. The reason for this is that the alignment of such sequence reads to one of the two reference sequences, e.g. the transgene or the HPC genome, would result in a rather long mismatch of sequence reads representing the nucleotide sequence of the other reference sequence, e.g. the HPC genome or the transgene, respectively, to which the sequence read was not aligned. For example, a sequence read mapping to both the transgene and the HPC genome would still, if aligned to the HPC genome, contain a rather large unmapped region that corresponds to the sequence of the transgene. Consequently, such sequence reads that span the boundaries between the transgene and the HPC genome are discarded by the alignment programs as low quality sequences with a high content of incorrect base pairs with respect to one of the two reference sequences (i.e. the HPC genome or the transgene sequence).

In a further embodiment, read 1 sequences of class 1 or 2 are combined with the corresponding read 2 sequences of respective class 4 or 3. The correct assignment of read pairs that belong together is achieved by a sequence identifier which is encoded in FastQ file generated for the sequence reads. The sequence identifier comprises information about the lane number on the flow cell, the tile number within the lane on which the respective sequence was attached to the flow cell, as well as the "x" and "y" coordinates of the nucleic acid cluster within the tile. Furthermore, the sequence identifier comprises an index number indicating the member of a paired-end sequencing pair (i.e. read 1 or read 2) (**FIGURE 2**).

In a further embodiment the respective read 1 and read 2 sequences of a read pair are separately aligned to the transgene sequence or to the HPC genome. Sequencing pairs in which read 1 maps to the transgene and read 2 maps to the host progenitor cell (HPC) genome (i.e. class 1/4 pairs) and sequencing pairs in which read 1 maps to the HPC genome and read 2 maps to the transgene (i.e. class 2/3 pairs) are kept for further analysis. Read pairs in which read 1 and read 2 sequences both map either to the transgene (e.g. class 1/3 pairs) or to the HPC genome (e.g. class 2/4 pairs) are not suitable for the identification of transgene insertion regions (TIRs) and are therefore discarded from the pool of sequence read pairs. This approach advantageously allows the identification of TIRs in the HPC genome without knowing the exact position of the transgene insertion site in the HPC genome. As explained above, conventional methods for NGS data analysis are not able to align sequence reads corresponding to overlapping regions of one or more separate reference genomes due to the large portion of resulting mismatches. For this reason, conventional single-end sequencing or conventional paired-end sequencing data processing is not suitable for the *de novo* identification of TIRs within a given HPC genome of known sequence. To achieve this goal, the present invention utilizes the information contained in the class 1/4 and class 2/3 read pairs obtained by the paired-end sequencing data processing approach described above. Sequence reads corresponding to class 1 and class 3 sequences, i.e. sequence reads that map to the known sequence of the transgene, are used to identify corresponding complement reads that map to the HPC genome, i.e. sequence reads corresponding to class 4 and class 3 sequences, respectively. Sequences that map to the HPC genome and that correspond to the class 1/4 and class 2/3 read pairs therefore represent regions within the HPC genomes that are adjacent to the boundaries of a given TIR because their complement read pair (either class 1 or class 3) maps to the transgene sequence. This approach to analysis of the paired-end sequencing data allows the identification of TIRs within an HPC genome which would otherwise be impossible when using methods commonly known in the art. As part of the inventive method, it is possible to identify transgene insertion regions in a given HPC genome and to compare TIRs within different samples derived from a given MCB, thereby determining the clonality of the MCB.

Thus, in a further embodiment of the present invention TIRs are identified by aligning the paired-end sequences corresponding to class 2 and class 4 to the HPC genome. The alignment of reads results in the identification of a 1000 nucleotide (1 kb)-long transgene insertion region within the HPC genome which is represented by class 2 and class 4 sequences of paired-end sequencing reads. To make the TIR prediction more reliable the region of transgene insertion is expanded to 2 kb starting from the center of the 1 kb region, extending the 1 kb region on both sides by 500 base pairs. The center of the 1 kb insertion region is defined as the nucleotide within the HPC genome with the highest sequence coverage. Considering a 2 kb insertion region of the transgene makes the prediction of TIRs within the HPC genome much more reliable because it allows for robust comparison of TIRs between different subclones even in the event that small variations in the sequence alignment of the one or more subclones occur.

It should be noted that alignment of class 2 and class 4 sequences to the HPC genome does not represent the exact position of the transgene insertion region in the HPC genome because the sequences corresponding to read 1 or read 2 of a paired-end sequencing pair usually span only 200 to 500 bp. The template to be sequenced, however, can be 800 bp or more in length. This difference can potentially lead to a gap, e.g. of 300 to 600 bp, in which the real transgene insertion site might be located. Thus, expanding the predicted transgene insertion region by an additional region of 500 bp on each site of the 1 kb region increases the credibility of the predicted TIRs (**FIGURE 3**).

In one embodiment of the invention, the identified TIRs in the random subclone (RSC), which was sequenced with higher sequence coverage compared to the sequence coverage of the remaining subclones (SCs), are analyzed for their sequence coverage and their number of reads variation to obtain one or more reference transgene insertion regions (RTIRs). RTIRs are selected based on the total number of sequence reads that map to a given position on the HPC genome. Within any given pool of TIRs, those TIRs TIRs that are represented by the highest sequence coverage are assigned as potential RTIRs. In addition to high sequence coverage, a further requirement for assignment as an RTIR is a low degree of overlap between the different read sequences representing a given TIR, i.e. the overlap between the read sequences representing a given TIR should be partial rather than identical, and the more partial the overlap, the more robust than predictable the identification of the TIR will be. Copies of sequences in the same region having a low degree of overlap are more likely to represent credible insertion region within the HPC genome as sequences that are characterized by large overlaps of their number of reads. Such "pile-up" of identical, i.e. coextensive, sequences are more likely due to sequencing artifacts that might have been introduced e.g. during library preparation and PCR amplification but do not represent reliable transgene insertion regions (**FIGURE 4**).

As mentioned above, the identification of credible RTIRs in the RSC genome is important as these RTIRs serve as the references for comparing the one or more subclones with the RSC. Incorrectly assigned RTIRs in the RSC might lead to an incorrect clonality assessment of the MCB because an incorrectly assigned RTIR in fact might be lacking in the genome of the one or more subclones (SCs) which are compared with the RSC. Thus, if an incorrectly assigned RTIR in the RSC genome has no corresponding CTIR in the one or more SC genomes, the evaluation of correspondence between the RSC and the one or more SCs might lead to the wrong conclusion that the one or more SCs are polyclonal because they lack a CTIR corresponding to a given RTIR, even though the latter is actually not a true transgene insertion region. For example, if a given TIR in the RSC would be incorrectly assigned as a RTIR due to low sequence coverage in the sequencing library of the RSC, such putative RTIR might not correspond to any of the one or more CTIRs in a respective SC genome. Consequently, the RSC and the respective SC would be (incorrectly) determined as being divergent and hence the MCB would be determined (also incorrectly) to be polyclonal.

In a further embodiment the first n RTIRs with the highest sequence coverage and lowest number of reads overlaps are determined, wherein n is an integer preferably from 5 to 50, for example 5, 10, 15, 20, 25, 30, 35, 40, 45, and 50 or any other single integer value between these. The number of RTIRs to be determined depends on various parameters, including the transfection method, the total number of TIRs, the HPC genome size, and the quality of sequencing data. Thus, the number of RTIRs which is required to determine clonality of a given Master Cell Bank (MCB) must be evaluated by the skilled person in a case-by-case manner. The number of required RTIRS is generally affected by: 1) The number of transgene inserted (e.g. lower is the number of insertion and lower will be the number of the RSC necessary for the analysis and 2) The number of reads for each RSC. In fact, in presence of a difficult DNA (meaning that the transgene are inserted in region of DNA difficult to sequence like telomeric regions) the number of reads characterizing each RSC will be very low meaning that the most robust RSCs will be lower than expected (e.g. 10 instead of 20). Generally, higher values of n will correlate to better statistical significance of the final clonality determination. For instance, the chance that the MCB arose from 2 different cells is smaller when 20 CTIRs in SCs are identical to corresponding RTIRs in an RSC, than when, for example, 5 CTIRs in SCs are identical to corresponding RTIRs in an RSC.

In a further embodiment of the present invention, the obtained reference transgene insertion regions (RTIRs) in the random subclone (RSC) are used as a basis of comparison with the corresponding comparative transgene insertion regions (CTIRs) in the one or more subclones (SCs) which have been independently expanded from the MCB and sequenced. Therefore, the genomic locations of CTIRs in each of the one or more SC genomes are compared with the corresponding genomic locations of RTIRs in the RSC genome.

In a further embodiment of the invention, the comparison of RTIRs in the RSC genome and corresponding CTIRs in the one or more SC genomes may be performed by generating a presence/absence matrix. In this matrix each insertion region may be represented by a binary color code indicating the presence or absence of a corresponding CTIR in a given SC genome with respect to a corresponding RTIR in the RSC genome. The RTIRs are represented by a first matrix dimension whereas the RSC and the one or more SCs are represented in a second, e.g. orthogonal, matrix dimension. As explained above, the presence or absence of a respective CTIR in a given SC genome relative to a respective RTIR in the RSC genome is represented in the matrix preferably as a binary color code, wherein a first color, e.g. black, represents the respective presence or absence of a respective RTIR in the RSC genome as well as the respective presence or absence of a respective CTIR in each of the SC genomes, and wherein a second color, e.g.white, represents the respective absence or presence of a respective RTIR in the RSC genome as well as the respective absence or presence of a respective CTIR in each of the SC genomes. This approach facilitates the easy optical comparison of RTIRs in the RSC genome with respective CTIRs in each of the SC genomes (**FIGURE 1(c)**). For instance, in a further embodiment, choosing black to represent correspondence of a CTIR with its corresponding RTIR and white to represent non-correspondence, a presence/absence matrix in which all matrix locations are black would indicate perfect correspondence between all CTIRs in each of the SCs and all RTIRs in the RSC, indicating moderate clonality of the MCB.

According to a further embodiment of the present invention the relationship between the random subclone (RSC) and each of the one or more subclones (SCs) may be evaluated by additional calculation of a distance matrix. In order to confirm the clonality, the expected distance is equal to 0. The additional calculation or, in other terms, the quantification of the distances, can help in the results interpretation. A "non-clonal" sample is expected to show a very low similarity (or very large distance) compared to "clonal" samples. The distance matrix may for example be calculated based on formula (I):

$$D_d (RSC, SC_m) = 1 - \left[ \frac{2 * N_{(total)}}{(N_{(CTIR)} + N_{(RTIR)})} \right]$$

Formula (I) represents the Dice coefficient, a commonly known statistical approach to compare the similarity of two samples. The distance function $D_d (RSC, SC_n)$ is calculated based on the Dice coefficient between the RSC genome and one of the m SC genomes. The variable $N_{(total)}$ of Formula (I) represents the total number of transgene insertion regions present both in the RSC genome and in one of the n SC genomes. The variable $N_{(CTIR)}$ represents the total number of transgene insertion regions present in one of the m SC genomes, whereas $N_{(RTIR)}$ represents the total number of reference transgene insertion regions in the RSC genome as determined in one of the above embodiments.

The calculated results of the distance function $D_d$ (RSC, $SC_m$) give information about the genetic distance, i.e. the genetic similarity or dissimilarity, between two individual samples on a scale of 0 to 1, wherein genetic and therefore clonal identity between two samples is represented by a distance of 0, with genetic and therefore clonal dissimilarity increasing above 0 to reach complete dissimilarity, i.e. no genetic relation whatsoever, at a calculated Dice coefficient of 1.

In a further embodiment of the invention the variables $N_{(total)}$, $N_{(CTIR)}$ and $N_{(RTIR)}$ of Formula (I) are calculated based on the data obtained by the presence/absence matrix of transgene insertion regions. The combination of an easy-to-view analysis, i.e. the representation of transgene insertion regions in a presence/absence matrix, with the similarity analysis based on the Dice coefficient provides an additional reliable, straight-forward and numerical approach for the evaluation of correspondence between the different subclones. In addition to the presence/absence matrix described above, similarity between the random subclone (RSC) and each of the one or more subclones (SCs) may further be analyzed by a distance matrix. The distance matrix is generated by transferring the data obtained by calculating the distance function $D_d$ based on the similarity between the RSC and each of the one or more SCs into a 2-dimensional coordinate system, in which the "y" axis represents the RSC and each of the one or more subclones and the "x" axis represents the distance of a particular sample with respect to the RSC. This 2-dimensional graph illustrates the distance between the RSC to itself and to each of the one or more SCs. If two or more samples are genetically identical, i.e. if the samples share the same transgene insertion regions, the distance between these two or more samples is "0". Otherwise, if the samples are not genetically identical, i.e. if the samples have different transgene insertion regions, then the distance between these samples is greater than "0", preferably "1". The distances of the one or more SCs with respect to the RSC are represented as dots in the distance matrix. Dots that superimpose on each other form a so called cluster. A cluster is a group of respective SC genomes having the same distance with respect to the RSC genome. Finally, the one or more respective SC genomes are considered to belong to the same cluster as the RSC genome if the distance between them as calculated according to Formula (I) is "0" (**FIGURE 1(c),** (129)).

According to a further embodiment of the present invention, the Master Cell Bank (MCB) for which clonality is to be determined is considered to be monoclonal based on the distance matrix if the random subclone (RSC) and the one or more SCs, preferably all SCs, are grouped into the same cluster (i.e. Dice coefficient = 0). In this case, the RSC genome and the one or more SC genomes, preferably all SC genomes evaluated, share the same transgene insertion regions, indicating that the RSC and the one or more SCs must have been expanded from a genetically identical pool of cells (MCB) that originated from a single MCB cell. In this case, the MCB may be considered monoclonal. This would mean that the MCB would be suitable for further production of the therapeutic protein encoded by the inserted transgene, in accordance with applicable regulatory standards.

In contrast, if one or more of the SCs evaluated deviate at one or more of their CTIRs from corresponding RTIRs in the RSC, and this deviation can be considered credible (i.e. not related to sequencing or PCR artifacts), then it may be safely assumed that the pool of cells (MCB) from which the RSC and the SCs derived did not originate from a single MCB cell, but rather multiple MCB cells. This may then be taken as proof that, despite limiting dilution to statistically result in one MCB cell per well in e.g. a 96-well plate, the well in question actually contained multiple, genetically heterogeneous cells (resulting from different random transgene insertions) or that the original single cell has through DNA replication and cell duplication lost certain insertion sites thus generating heterogeneity of the MCB. In this case, one may conclude that the MCB in question was not monoclonal, but rather polyclonal or has become polyclonal due to genomic instability. This would mean that the MCB would not be suitable for further production of the therapeutic protein encoded by the inserted transgene, in accordance with applicable regulatory standards and that, accordingly, a new monoclonal MCB must be identified for producing the protein of interest from the inserted transgene.

All the methods and embodiments described above also apply to single cell. In such a case the Single Cell Sequencing protocol is performed. The difference between the single cell sequencing and the sequencing of MCB or subclones is only related to the way the DNA is extracted (see example 9). The methods and embodiments are not fully repeated for conciseness. Simply, some adjustments in the wording have to be introduced: when applied to a method for the identification of the transgene insertion sites on single cell, the terms RSC and HCP are changed in RSgC (for Reference single cell) and the terms SC and MCB are changed in SgC (single cell).

In short the present disclosure also discloses a method for the identification of the transgene insertion sites on single cell, said single cell resulting from predictable or not predictable insertion of a transgene of known sequence into a reference single cell (RSgC) genome of known sequence, said method comprising the steps of:

a) Identifying one or more transgene insertion regions (TIRs) in the genome of a single cell (SgC), wherein the SgC has been isolated and wherein said identifying is achieved by paired-end sequencing of said SgC genome to obtain an SgC genome sequence or SgC genome sequences; and alignment of said SgC genome sequence or sequences to said known RSgC genome sequence and said known transgene sequence, thereby yielding one or more transgene insertion regions (TIRs);

b) Determining one or more TIRs as identified in step (a) with the highest degree of sequence coverage, wherein said sequence coverage refers to the number of times a given nucleic acid sequence containing a given TIR is read during the sequencing process by partially overlapping reads; wherein said one or more TIRs with the highest degree of sequence coverage are assigned as reference TIRs (RTIRs).

Preferably, the paired-end sequencing involves sequencing of a given nucleic acid molecule from both ends of said nucleic acid molecule, thereby generating pairs of reads for a given nucleic acid molecule representing a fragment of the genome to be sequenced. Also preferably, the SgC results from the insertion of said transgene at multiple positions into said RSgC genome, wherein said random insertion is preferably effected using a retroviral vector. In said method, the determination of TIRs comprises classification of paired-end read 1 sequences and paired-end read 2 sequences derived from paired-end libraries into 4 classes, wherein

- class 1 comprises read 1 sequences mapping to said transgene;
- class 2 comprises read 1 sequences mapping to said RSgC genome;
- class 3 comprises read 2 sequences mapping to said transgene; and
- class 4 comprises read 2 sequences mapping to said RSgC genome;

wherein said read 1 and said read 2 represent respective forward and backward reads corresponding to the 5' and 3' ends of a given nucleic acid molecule within a nucleic acid cluster generated in sequencing of a nucleic acid library of said RSgC or said one or more SgCs. Preferably, read 1 sequences are combined with the corresponding read 2 sequences using a flow cell sequence identifier, wherein said sequence identifier comprises information of the flow cell lane, the tile number within the flow cell, the "x" coordinate of the nucleic acid cluster within a tile, and the "y" coordinate of the nucleic acid cluster within a tile, thereby assigning each sequence pair corresponding to read 1 and read 2 sequences a unique position within the flow cell. Also preferably, the respective read 1 and read 2 sequences of a respective read pair are separately aligned to the known sequences of the transgene and the RSgC genome. Even preferably, only the read pairs comprising class 1 and 4 sequences and the read pairs comprising class 2 and class 3 sequences are selected for further analysis. Even more preferably, TIRs are identified by aligning the paired-end read sequences corresponding to class 2 and class 4 to the RSgC genome, thereby defining a 2kb region for each of said TIRs in the RSgC genome. In summary, the present inventors provide a novel method for confirming the clonality of an MCB, avoiding the traditionally time- and labor-intensive testing of the MCB required by health authorities for approving the MCB as a production cell line in the manufacture of proteins intended for pharmaceutical application. The present method benefits from exact reproducibility, which allows the robust clonality assessment of a MCB. The inventors achieve this beneficial effect by a novel combined approach of paired-end sequencing and subsequent bioinformatic data processing of the sequencing data obtained. In particular, the novel approach to processing the paired-end sequencing data allows identification of transgene insertion regions in a known sequence of an HPC genome *de novo,* which is not possible by conventional single-read sequencing or conventional processing of paired-end sequencing data. The present disclosure thus provides powerful means for robustly assessing the quality of an MCB for use in the production of protein products intended for pharmaceutical application.

It also provides a novel method for the identification of the transgene insertion sites on single cell, based on the same approach as for assessing the clonality of a MCB.

The following examples, including the experiments conducted and the results achieved, are provided for illustrative purposes only and are not construed as limiting the present invention.

## EXAMPLES

Example 1: Clone selection and Subcloninq

The MCB, the clonality of which is to be assessed, was generated by transfection of two transgenes carrying light and heavy chain into the genome of a Chinese hamster ovary (CHO) cell line that served as the host progenitor cell (HPC). The transfection was performed by Gala@ (Catalent) using the GPEx® technology and a single limiting dilution was performed in order to obtain the monoclonal cell line (See "GPEx®: a flexible method for the rapid generation of stable, high expressing, antibody producing mammalian cell lines", Gregory T. Beck, Book: Current Trends in Monoclonal Antibody Development and Manufacturing, Publisher: Springer New York, 2010). In order to investigate the clonality of this cell line 25 subclones (SCs) where generated by dilution of the MCB. The limiting dilution was performed as follows. The MCB was thawed at room temperature and then incubated in a T 75 flask with 20 mL of DMEM with 10% FBS for 24 hours at 37°C, 5% $CO_2$. The next day the medium was removed, cells were detached with trypsin and resuspended in fresh medium for cell counting. Limiting dilutions were then performed to reach a concentration of 5 cells/ml. The dilution was then plated into a 96 well plate, placing 100 $\mu$L in each well in order to theoretically obtain 0.5 cells per well. Here, it was understood that some of the wells may contain no cells, but given the low cell/well ratio, there is a high probability that if a well contained a cell at all, it would contain no more than one cell. The plate was then incubated at 37°C, 5% $CO_2$ for 24 hours. The next day the 96 well plate was analyzed under the microscope and wells containing one cell per well were marked. The plate was further incubated in order to obtain the cells at a confluent state which were previously marked. Cells that reached confluency were selected as subclones for further analysis.

Independently, a divergent MCB ($MCB_\Delta$) was used. The divergent $MCB_\Delta$ resulted from independent transfection of the host progenitor cell with the transgene, and was for intended use as a negative control, providing a cell known from the outset to be genetically different than the MCB. The $MCB_\Delta$ is thus expected to have no transgene insertion regions in common with the subclones or the MCB for which clonality is to be determined. The MCB and divergent $MCB_\Delta$ the serve as respective positive and negative controls for the purpose of evaluating the claimed method. In practice, determination of MCB clonality can also be assessed in the absence of control samples based on the clonality assessment of the subclones derived from the MCB.

Example 2: DNA extraction

DNA extraction from the 25 subclones, the MCB and the divergent $MCB_\Delta$ was performed on an affinity column using the QIAamp Blood DNA Mini kit (QIAGEN) according to the manufacturer's and internal working instructions. Briefly, cell pellets were resuspended in phosphate buffered saline (PBS) according to the sample concentration and split into different aliquots. 200 $\mu$L of lysis buffer and 20 $\mu$L of proteinase K were added to each sample. Samples were mixed thoroughly by vortexing, and incubated at 56°C for 10 minutes. Then, 200 $\mu$L ethanol (96 to 100%) was added and the mixture was transferred into the DNeasy Mini spin column (QIAGEN) placed in a 2 mL collection tube. Samples were washed and centrifuged for a minute at 13,000 rpm to remove any residual ethanol. Elution was performed with 150 $\mu$L of water directly added to the DNeasy membrane (QIAGEN). Eluates of the same clone where combined.

Each sample was incubated with RNase enzyme (Roche) at 37°C for 30 minutes to remove any residual RNA. After incubation samples were quantified by NanoDrop® ND-1000 spectrophotometer and the absorbance ratio 260/280 evaluated to assess DNA quality.

Example 3: Ilumina library preparation and sequencing

Library preparation for the 25 subclones, the MCB and the $MCB_\Delta$ was performed using the TruSeq DNA kit (Illumina) according to manufacturer's instructions. Briefly, 2.6 $\mu$g of each subclone DNA were fragmented by the Covaris S220 instrument to obtain 300 bp dsDNA fragments with 3' or 5' overhangs. Overhangs were converted enzymatically into blunt ends. A single adenine (A) nucleotide was added to the 3' ends of the blunt fragments to prepare fragments for adapter ligation. The ligation of multiple indexing adapters to the ends of the DNA fragments allows the hybridization onto a flow cell. Selective enrichment of those DNA fragments that have adapter molecules on both ends was performed to increase library yield.

The quality of DNA libraries was analyzed by an Agilent 2100 Bioanalyzer to validate the mean size of the fragments in the DNA libraries. Libraries were further quantified by Fluorometer Qubit® 2.0.

DNA library clusters were generated on the Illumina cBot instrument (Illumina, TruSeq PE Cluster Kit v3 cBot HS kit) according to manufacturer's instruction. Sequencing was performed in paired-end mode (2x100 cycles) by using the Illumina HiSeq 1000 instrument. The sequencing was performed using the TruSeq SBS Kit v3-HS-200-cycles kit (Illumina®). Samples were loaded onto a flow cell v3. The 25 subclones, the MCB and the divergent $MCB_\Delta$ were each loaded onto a separate lane. Subclone # 25 ($SC_{25}$) was loaded onto 3 lanes to obtain higher sequencing coverage. It was intended to use $SC_{25}$ as the random subclone (RSC) in which the identified TIRs with the highest degree of partially overlapping sequence coverage would be identified as reference TIRs (RTIRs). For this reason, it was desired to maximize sequence coverage for $SC_{25}$ to ensure reliable identification of the RTIRs. Redundant loading and subsequent sequencing of DNA fragments derived from $SC_{25}$ was conducive to this end.

All the samples were sequenced on Illumina HiSeq1000. For each sample at least 170 million of 2x100bp reads were obtained. The PHRED quality score for each sample analyzed was higher than 70%. Results are summarized in **Table 1.** The mean coverage for each sample sequenced was at least 16X (considering the total size of the CHO genome to be 2.4 Gb). The coverage for the $SC_{25}$ instead was around 50X due to sequencing of the $SC_{25}$ library on 3 lanes, while the remaining libraries were sequenced on one lane (see **Table 1**).

The raw data were then further processed with CASAVA V. 1.8.2 (Illumina) in order to convert the basecall files (.bcl) into FastQ files. The FastQ files are text files containing the nucleotide sequence of the reads and the relative quality scores for each base pair. The obtained FastQ files were then processed by the bioinformatic pipeline that generate a binary (.bam) file for each sample containing all the reads mapping to the CHO reference genome, including their coordinates on the reference genome

Example 4: Bioinformatical analysis

For the analysis of the MCB, the 25 subclones and the divergent MCB$_\Delta$, different bioinformatic approaches were applied in order to detect the boundaries of the transgenes randomly inserted into the CHO genome. For the boundaries detection the concept described in Figure 1B was used. Once selected, boundaries were analyzed by means of statistical tools, see for instance the statistical approach described in Formula I.

The paired-end sequencing performed was used considering separate read 1 and read 2 sequences. The bioinformatical analysis was performed as follows: read 1 and read 2 were mapped separately to the transgene sequence of the CHO genome of known sequence (corresponding to the host progenitor cell (HPC) genome) using the Burrows-Wheeler Aligner (BWA) V. 0.6.1-r104 (Li et al. (2009). Fast and accurate short read alignment with Burrows Wheeler transform. Bioinformatics; 25(14): 1754-60). Once mapped, four kinds of files were obtained: read 1 mapped to the transgene sequence (class 1 sequence), read 1 mapped to the CHO genome (class 2 sequence), read 2 mapped to the transgene sequence (class 3 sequence) and read 2 mapped on the CHO genome (class 4 sequence) (**FIGURE 1(b)**).

For read 1 and read 2 mapping on the transgene (respective classes 1 and 3) a list of the reads was created. The corresponding "paired" read was then searched in the read 1 and read 2 sequences mapping to the CHO genome (respective classes 2 and 4) by the Illumina sequence identifier (**FIGURE 2**). These reads were mapped onto the CHO reference genome by Burrows-Wheeler Aligner (BWA) V. 0.6.1-r104.

Those reads which aligned to the CHO represent regions adjacent to the boundaries of the transgene inserted in the CHO genome because they have the complement read pair mapping to the transgene. Finally, the insertion regions were identified based on position on the different scaffold of the CHO reference genome by means of Geneious® software V. 6.0 (this software allows an easy-to-view identification of the TIR at highest coverage, and thus simplifies the visualisation of the results)

After identification of the transgene insertion regions in all samples, a statistical analysis approach was performed to determine one or more reference transgene insertion regions (RTIRs). This selection was performed on the basis of two characteristics: (1) the number of reads representative for each insertion region and (2) the degree of overlap of these reads in the area of the insertion region (**FIGURE 4**). The combination of these two parameters was important to identify the insertion sites with the highest coverage while at the same time avoiding aberrant read pile-ups due to biases resulting from PCR and/or library preparation.

As mentioned above, the selection of RTIRs was performed on SC$_{25}$, and this was the reason that SC$_{25}$ was sequenced with higher sequence coverage (3 Flow Cell Ln. redundancy as explained above). The alignment file of this sample was opened with Geneious® software and the first 20 TIRs that fulfill the above requirements were determined and, due to fulfilling these requirements, were designated as RTIRs. These 20 RTIRs thus represented the most reliable transgene insertion regions (TIRs) and were therefore used as a basis for comparison in subsequent statistical analysis (position of the RTIRs not shown).

Example 5: Comparison of the insertion regions

A presence/absence matrix was created to provide a comparison of the selected RTIRs among all the samples. The presence/absence matrix was modeled after an electrophoresis gel, with the "bands" representing the intersection between RTIRs in SC$_{25}$ (corresponding to the RSC) and corresponding CTIRs in a given SC. A The presence/absence of a given RTIR as a CTIR in a given SC was indicated by a binary code pattern, representing the presence (1, black) or absence (0, white) of a given CTIR. All samples were then compared to each other for all the insertion regions identified (**FIGURES 1(c) and 5**).

In theory the basis of this analysis relates to the mechanism of the GPEx® system to randomly insert the transgene into HPC genome at a plurality of position. In fact, if the MCB, for which clonality is to be determined, is monoclonal the transgene insertion regions (TIRs) randomly inserted into the HPC genome should be identical between the 25 subclones and the MCB, while the divergent MCB$_\Delta$ should not have any insertion site in common with the subclones and the MCB. For this reason, the presence/absence matrix was designed to deliver information relating to the presence/absence of each reference transgene insertion region (RTIR) in each of the subclones (**FIGURE 5**). The results indicate that almost all subclones and the MCB share the same transgene insertion regions with respect to the RTIRs in the random subclone (RSC) genome, i.e. SC$_{25}$. In contrast, TIRs of the divergent MCB$_\Delta$ showed a different result in which none of the 20 RTIRs was present.

Furthermore, the presence/absence matrix showed that for two samples one transgene insertion region was apparently missing. In particular, RTIR$_{18}$ in SC$_4$ and RTIR$_1$ in SC$_{24}$ were not observed (**FIGURE** 5). Further investigation was carried out on these two samples by PCR and traditional Sanger sequencing. To this end, two sets of specific primers were designed to amplify RTIR$_1$ and RTIR$_{18}$ in respective SC$_4$ and SC$_{24}$. PCR reactions were performed on SC$_4$ and SC$_{24}$ using the specific sets of primers for RTIR$_1$ and RTIR$_{18}$. Furthermore, SC$_1$ and the CHO host cell DNA (that does not contain any TIR) were used as respective positive and negative controls.

PCR products for $RTIR_1$ and $RTIR_{18}$ were observed in both samples of $SC_4$ and $SC_{24}$ as well as in the positive control of $SC_1$. No PCR product was observed in the negative control sample. The PCR products of respective RTIRs were purified and the purified templates were sequenced on an ABI Prism 3130 sequencing platform. The results of the Sanger sequencing indicate that for both $RTIR_1$ and $RTIR_{18}$ $SC_4$ as well as $SC_{24}$ were positive indicating that also in these two samples $RTIR_1$ and $RTIR_{18}$ were present (data not shown).

Example 6: Similarity analysis

Similarity among the samples was expressed numerically by using a cluster analysis approach. Specifically, the distance between the MCB and each of the 25 subclones as well as the divergent $MCB_\Delta$ was calculated using the Dice coefficient based on following formula :

$$D_d(A, B) = 1 - \left[ \frac{2 * N_{(total)}}{(N_{(A)} + N_{(B)})} \right]$$

$D_d$ (A, B) represents the distance function between two samples A and B, wherein $N_{(total)}$ is the number of insertion regions present both in both samples A and B; $N_{(A)}$ is the total number of insertion regions present in sample A; and $N_{(B)}$ is the total number of insertion regions present in sample B; wherein $D_d$ (A, B) represents the distance, on a scale of 0 to 1, wherein a distance of 0 represents clonal identity between said RSC and a respective $SC_n$, and 1 represents clonal difference.

In order to graphically show the distance among all the samples, a MultiDimensional Scaling (MDS) approach was used (FIGURE 6)(Kruskal and Wish (1978), Multidimensional Scaling, Sage University Paper series on Quantitative Application in the Social Sciences, 07-011, Beverly Hills and London, Sage Publications; Michael R. Anderberg (1973) Cluster analysis for applications, Academic Press, New York)

The results of the similarity analysis indicate that two distinct subgroups were obtained. The first subgroup corresponds to the 25 subclones and the MCB whereas the second subgroup corresponds to the divergent $MCB_\Delta$. The distance between these two subgroups was 1 or 100%, indicating that all the 25 subclones and the MCB correspond to the same subgroup (cluster) whereas as the divergent MCB correspond to a different cluster (subclone) (**FIGURE 6**).

Example 7: Probability analysis

In order to assess the probability that two samples from different populations, i.e. having different transgene insertion regions, share the same 20 RTIRs, an experimental calculation was performed based on the following formula:

$$p(1) = \frac{1}{M} * S$$

wherein M is the number of possible transgene insertion regions and S is the number of reads of the transfected transgene.

The CHO genome is 2.4 Gb long. Based on the applied technology of combined NGS analysis and bioinformatical data processing the transgene insertion regions can be determined to a resolution of 2kb within the CHO genome. Based on the assumption that the technology can potentially identify 1.2 million of possible transgene insertion regions within the CHO gemone (2.4 Gb/2kb) and the assumed transfection rate of 700 copies of the transgene into the CHO progenitor cell, a probability of p(1) $0 \sim 10^{-30}$ is obtained.

This result indicates that the likelihood that two subclones derived from different MCB populations share common transgene insertion regions tends to be 0. In practice, the question of determining clonality of a MCB will often be a binary determination , meaning that subclones are either 100% identical to each other, i.e. having all insertion regions in common, or will have 0% or a very low fraction of (probably coincidental) transgene insertion regions in common. Generally, a determination of MCB monoclonality will typically require 100% genetic congruence between RTIRs in the RSC, and all CTIRs in each of the subclones evaluated. Any individual divergences between a given RTIR and its corresponding CTIR in a given subclone may be more closely evaluated using alternative sequencing methodology, e.g. Sanger sequencing.

The probability was calculated taking into account different biological aspects: 1) theoretically, retrovectors insert the transgene randomly into the DNA (several works however demonstrate that there are some particular areas of the target genome that retrovirus (and retrovectors) prefers for the insertion), and 2) the insertion of the GOIs is also related to the kind of retrovector used for the transfection (Bushman et al. Genome-wide analysis of retroviral DNA integration. Nat Rev Microbiol 2005;3(11):848-858; Felice et al. Transcription factor binding sites are genetic determinants of retroviral integration in the human genome. PLoS ONE 2009;4(2):e4571.). For example, recently, studies showed that MLV-derived vectors integrate preferentially within or around genes involved in cell regulation such as transcription start sites, enhancers or promoters. In addition seems that the accessibility of the target DNA plays an important role in the integration of the transgene (e.g centromeric heterochromatin regions seems to be less favoured for the integration) (LaFavey et al., MLV integration site selection is driven by strong enhancers and active promoters Nucleic Acids Research, 2014, Vol. 42, No. 7 4257-4269. For these reasons, the insertion by the retroviral vector was defined as non-totally-random and the appropriate probability approach was applied.

Example 8: confirmation of the feasibility of the methods

The methods described in Examples 1 to 7 have been applied to a second type of MCB, expressing a different monoclonal antibody (mAb2). This second MCB was generated by transfection of a transgene expressing the light and heavy chains of mAb2 into the genome of a CHO cell line that served as HPC. It was possible to assess clonality of this MCB expressing mAb2 according to the methods of the invention (data not shown), confirming that the methods according to the invention are reproducible whatever the transgene is, and possibly whatever the cell line is.

Example 9: Single cell analysis

The method described for the identification of the transgene insertion sites can be applied on single cell in case the Single Cell Sequencing protocol is performed. The difference between the single cell sequencing and the sequencing of MCB or subclones is only related to the way the DNA is extracted. In case of Single Cell Sequencing in fact the cells are subjected to Whole Genome Amplification to obtain a sufficient amount of DNA. On the contrary the DNA extraction of MCB and sublcones can be performed with traditional DNA extraction methods described in

example 2

Table 1: Summary of the most important quality parameters generated by the HiSeq 1000 instrument, with parentheses indicating acceptance criteria for each value.

| Sample | Sequencing lane | Cluster density (K/mm2) (66-1200 K/mm2) | Phasing/ Prephasing (<0.6) | Reads (M) (>150 M) | Reads PF (>130 M) | Phred Scores Q>30 (>70%) |
|---|---|---|---|---|---|---|
| SC #1 | 1 | 1130 +/- 66 | 0.180 / 0.197 | 312.3 | 247.33 | 87.8 |
| SC #2 | 2 | 1147 +/- 66 | 0.172 / 0.195 | 317.09 | 247.49 | 87.7 |
| SC #3 | 3 | 1100 +/- 66 | 0.170 / 0.207 | 304.01 | 260.58 | 89.6 |
| SC #4 | 4 | 1141 +/- 72 | 0.169 / 0.214 | 315.5 | 252.4 | 88.3 |
| SC #5 | 5 | 1062 +/- 91 | 0.174 / 0.200 | 293.74 | 255.27 | 90.2 |
| SC #6 | 6 | 1100 +/- 58 | 0.171 / 0.196 | 304.02 | 257.01 | 89.5 |
| SC #7 | 7 | 1115 +/- 62 | 0.163 / 0.205 | 308.17 | 255.72 | 88.7 |
| SC #8 | 8 | 1157 +/- 48 | 0.172 / 0.198 | 319.81 | 251.33 | 87.9 |
| SC #9 | 1 | 1000 +/- 113 | 0.206 / 0.727 | 276.46 | 252.67 | 83.6 |
| SC #10 | 2 | 935 +/- 116 | 0.197 / 0.529 | 258.59 | 238.9 | 89.6 |
| SC #11 | 3 | 893 +/- 112 | 0.195 / 0.637 | 257.02 | 228.4 | 88.1 |
| SC #12 | 4 | 850 +/- 132 | 0.208 / 0.784 | 235.09 | 217.38 | 83.5 |
| SC #13 | 5 | 707 +/- 103 | 0.217 / 0.712 | 195.56 | 185.78 | 87.3 |
| SC #14 | 6 | 813 +/- 106 | 0.204 / 0.679 | 224.71 | 211.38 | 86.7 |
| SC #15 | 7 | 657 +/- 92 | 0.210 / 0.703 | 181.53 | 172.69 | 86.3 |

(continued)

| Sample | Sequencing lane | Cluster density (K/mm2) (66-1200 K/mm2) | Phasing/ Prephasing (<0.6) | Reads (M) (>150 M) | Reads PF (>130 M) | Phred Scores Q>30 (>70%) |
|--------|-----------------|-------------------------------------------|------------------------------|----------------------|---------------------|----------------------------|
| SC #16 | 8 | 729 +/- 88 | 0.213 / 0.811 | 201.54 | 188.6 | 80 |
| SC #17 | 1 | 876 +/- 84 | 0.221 / 1,040 | 242.07 | 225.71 | 70.6 |
| SC #18 | 2 | 803 +/- 101 | 0.192 / 0.663 | 222.12 | 209.61 | 88.6 |
| SC #19 | 3 | 666 +/- 101 | 0.209 / 0.759 | 184.16 | 174.7 | 85.6 |
| SC #20 | 4 | 873 +/- 102 | 0.219 / 0.993 | 241.39 | 223.91 | 73.5 |
| SC #21 | 5 | 1034 +/- 82 | 0.203 / 0.772 | 285.86 | 256.2 | 80.5 |
| SC #22 | 6 | 704 +/- 92 | 0.193 / 0.608 | 194.72 | 185.65 | 91 |
| SC #23 | 7 | 907 +/- 98 | 0.193 / 0.569 | 250.9 | 231.14 | 88.4 |
| SC #24 | 8 | 747 +/- 97 | 0.199 / 0.674 | 206.41 | 195.72 | 87.9 |
| SC #25 | 6 | 807 +/- 116 | 0.166 / 0.277 | 223.2 | 211.47 | 94.1 |
| SC #25 | 7 | 819 +/- 106 | 0.168 / 0.271 | 226.5 | 214.28 | 94 |
| SC #25 | 8 | 1006 +/- 113 | 0.172 / 0.310 | 278.16 | 253.77 | 91.5 |
| MCB | 1 | 883 +/- 100 | 0.173 / 0.356 | 244.22 | 228.27 | 93 |
| divergent MCB | 2 | 1032 +/- 84 | 0.165 / 0.327 | 285.31 | 256.07 | 91 |

## Claims

1. A method for determining the clonality of a Master Cell Bank (MCB), said MCB resulting from predictable or not predictable insertion of a transgene of known sequence into a host progenitor cell (HPC) genome of known sequence, said method comprising the steps of:

   a) Identifying one or more transgene insertion regions (TIRs) in the genome of a reference subclone cell (RSC), wherein the RSC has been isolated from the MCB for which clonality is to be determined, and wherein said identifying is achieved by

   i. paired-end sequencing of said RSC genome to obtain an RSC genome sequence or RSC genome sequences; and
   ii. alignment of said RSC genome sequence or sequences to said known HPC genome sequence and said known transgene sequence,

   thereby yielding one or more transgene insertion regions (TIRs);
   b) Determining one or more TIRs as identified in step (a) with the highest degree of sequence coverage, wherein said sequence coverage refers to the number of times a given nucleic acid sequence containing a given TIR is read during the sequencing process by partially overlapping reads;
   wherein said one or more TIRs with the highest degree of sequence coverage are assigned as reference TIRs (RTIRs);
   c) Identifying one or more transgene insertion regions (TIRs) in the respective genomes of one or more subclone (SCs);
   wherein each of the SCs has been isolated from the MCB for which clonality is to be determined but is independent of said RSC,
   wherein said identifying is achieved by

   i. paired-end sequencing of each respective SC genome to obtain an SC genome sequence or SC genome sequences; and

ii. alignment of each respective SC genome sequence or sequences to said known HPC genome sequence and said known transgene sequence,

thereby yielding one or more comparative transgene insertion regions (CTIRs);

d) Comparing said one or more RTIRs determined in step (b) with the respective CTIRs determined in step (c);

e) Evaluating the correspondence between each of said one or more CTIRs present in a respective SC and corresponding RTIRs present in said RSC, wherein the relationship between said RSC and each of the said one or more SCs is evaluated by calculation of a distance matrix; and

f) Determining clonality of said MCB based on said correspondence evaluated in part (e), wherein said MCB is considered to be monoclonal, if said RSC and said one or more SCs are grouped into the same cluster, and wherein the distance matrix is calculated based on the following formula (I),

$$D_d (RSC, SC_m) = 1 - (2^* N_{(total)} / [N_{(CTIR)} + N_{(RTIR)}])$$

wherein $D_d (RSC, SC_m)$ represents the distance function between said RSC genome and a respective $SC_m$ genome, wherein $N_{(total)}$ is the number of insertion regions present both in said RSC genome and said $SC_m$ genome; $N_{(CTIR)}$ is the total number of insertion regions present in said $SC_m$ genome; and $N_{(RTIR)}$ is the total number of insertion regions present in said RSC genome; wherein $D_d (RSC, SC_m)$ represents the distance, on a scale of 0 to 1, wherein a distance of 0 represents clonal identity between said RSC and a respective $SC_m$, and 1 represents clonal difference.

2. The method of claim 1, wherein paired-end sequencing involves sequencing of a given nucleic acid molecule from both ends of said nucleic acid molecule, thereby generating pairs of reads for a given nucleic acid molecule representing a fragment of the genome to be sequenced.

3. The method of claim 1 or 2, wherein said RSC is sequenced with a higher sequence coverage compared to said one or more SCs.

4. The method of any of the above claims, wherein said MCB results from the insertion of said transgene at multiple positions into said HPC genome, wherein said random insertion is preferably effected using a retroviral vector.

5. The method of any of the previous claims, wherein the determination TIRs comprises classification of paired-end read 1 sequences and paired-end read 2 sequences derived from paired-end libraries into 4 classes, wherein

• class 1 comprises read 1 sequences mapping to said transgene;
• class 2 comprises read 1 sequences mapping to said HPC genome;
• class 3 comprises read 2 sequences mapping to said transgene; and
• class 4 comprises read 2 sequences mapping to said HPC genome;

wherein said read 1 and said read 2 represent respective forward and backward reads corresponding to the 5' and 3' ends of a given nucleic acid molecule within a nucleic acid cluster generated in sequencing of a nucleic acid library of said RSC or said one or more SCs.

6. The method of claim 5, wherein read 1 sequences are combined with the corresponding read 2 sequences using a flow cell sequence identifier, wherein said sequence identifier comprises information of the flow cell lane, the tile number within the flow cell, the "x" coordinate of the nucleic acid cluster within a tile, and the "y" coordinate of the nucleic acid cluster within a tile, thereby assigning each sequence pair corresponding to read 1 and read 2 sequences a unique position within the flow cell.

7. The method of claims 5 and 6, wherein the respective read 1 and read 2 sequences of a respective read pair are separately aligned to the known sequences of the transgene and the HPC genome.

8. The method of any of claims 5 to 7, wherein only the read pairs comprising class 1 and 4 sequences and the read pairs comprising class 2 and class 3 sequences are selected for further analysis.

9. The method of any of claims 5 to 8, wherein said TIRs are identified by aligning the paired-end read sequences corresponding to class 2 and class 4 to the HPC genome, thereby defining a 2kb region for each of said TIRs in the HPC genome.

10. The method of any of the previous claims, comprising determining n RTIRs with the highest sequence coverage in the paired-end NGS library; wherein n is an integer from 5 to 50, preferably 5, 10, 15, 20, 25, 30, 35, 40, 45 or 50.

11. The method of claim 10, wherein the first n RTIRs with highest sequence coverage are determined based on

a) the number of reads of a respective paired-end read sequence corresponding to class 2 and class 4 mapping to the HPC genome, wherein higher number of reads indicates inclusion as an RTIR; and
b) the partial overlap of the number of reads of a respective paired-end read sequence corresponding to class 2 and class 4, wherein lower partial overlap of number of reads indicates inclusion as an RTIR.

12. The method of claim 10 or 11, wherein each of the first n RTIRs in said RSC genome is compared with the corresponding genomic location of said CTIRs in each of said one or more SC genomes.

13. The method of claim 12, wherein comparison of said RTIRs in said RSC and said CTIRs in said one or more SCs is achieved by generating a presence/absence matrix of insertion regions, wherein one matrix dimension represents said n RTIRs of said transgene in said RSC genome and another, preferably orthogonal, matrix dimension represents said RSC and each of said one or more SCs.

14. The method of claim 13, wherein the presence or absence of a respective CTIR in said one or more SCs relative to a respective RTIR in said RSC is represented in the matrix as a binary color code, wherein a first color represents the respective presence or absence of a respective RTIR in said RSC, the respective presence or absence of a respective CTIR in said one or more SCs, and wherein a second color represents the respective absence or presence of a respective RTIR in said RSC, the respective absence or presence of a respective CTIR in said one or more SCs.

15. The method of any of the preceding claims, wherein the parameters $N_{(total)}$, $N_{(CTIR)}$ and/or $N_{(RTIR)}$ are calculated based on the presence/absence matrix of insertion regions generated according to either of claims 1 or 14.

16. The method of any of the preceding claims, wherein the method comprises representing said one or more SCs relative to the RSC on a common distance matrix.

17. The method of claim 16, wherein two respective genomes are considered to belong to a common cluster if the distance between them as calculated according to Formula (I) is 0.

**Patentansprüche**

1. Verfahren zur Bestimmung der Klonalität einer Master-Zell-Bank (MCB), wobei die MCB aus einer vorhersagbaren oder nicht vorhersagbaren Insertion eines Transgens einer bekannten Sequenz in ein Wirtsvorläuferzell (HPC)-Genom einer bekannten Sequenz resultiert, wobei das Verfahren die folgenden Schritte umfasst:

a) Identifizierung einer oder mehrerer Transgen-Insertions-Regionen (TIRs) im Genom einer Referenz-Subklon-Zelle (RSC), wobei die RSC aus der MCB, für welche Klonalität bestimmt wurde, isoliert wurde und wobei die Identifikation erreicht wird durch

i. Paired-End-Sequenzierung des RSC-Genoms, um eine RSC-Genomsequenz oder RSC-Genomsequenzen zu erhalten; und
ii. Alignment der RSC-Genomsequenz oder -sequenzen mit der bekannten HCP-Genomsequenz und der bekannten Transgensequenz,

was zu einer oder mehreren Transgen-Insertions-Regionen (TIRs) führt;
b) Bestimmung einer oder mehrerer TIRs, wie in Schritt (a) identifiziert, mit dem höchsten Grad an Sequenzabdeckung,
wobei sich die Sequenzabdeckung auf die Anzahl der Lesevorgänge mit denen eine gegebene Nucleinsäuresequenz, welche ein gegebenes TIR enthält, während des Sequenziervorgangs durch teilweise überlappende Lesevorgänge bestimmt wurde, bezieht;
wobei die eine oder mehreren TIRs mit dem höchsten Grad an Sequenzabdeckung als Referenz-TIRs verwendet werden (RTIRs);

c) Identifizierung einer oder mehrerer Transgen-Insertions-Regionen (TIRs) in den entsprechenden Genomen eines Subklons oder mehrerer Subklone (SCs);

wobei jeder der SCs aus der MCB, für welche die Klonalität zu bestimmen ist, aber welcher unabhängig von dem RSC ist, isoliert wurde,

wobei die Identifizierung erreicht wird durch

i. Paired-End-Sequenzierung jedes entsprechenden SC-Genoms, um eine SC-Genomsequenz oder SC-Genomsequenzen zu erhalten, und

ii. Alignment jeder entsprechenden SC-Genomsequenz oder -sequenzen mit der bekannten HPC-Genomsequenz und der bekannten Transgensequenz, wodurch eine oder mehrere Vergleichs-Transgen-Insertions-Regionen (CTIRs) erhalten werden;

d) Vergleich der einen oder mehreren RTIRs, welche in Schritt (b) bestimmt wurden, mit den entsprechenden CTIRs, welche in Schritt (c) bestimmt wurden;

e) Evaluierung der Entsprechung zwischen jeder der einen oder mehreren CTIRs, welche in einem entsprechenden SC anwesend sind, und korrespondierenden RTIRs, die im RSC anwesend sind, wobei die Beziehung zwischen dem RSC und jedem des einen oder der mehreren SCs durch die Errechnung einer Distanzmatrix evaluiert wird; und

f) Bestimmung der Klonalität der MCB basierend auf der Entsprechung, welche in Teil (e) evaluiert wurde,

wobei die MCB als monoklonal verstanden wird, wenn der RSC und der eine oder die mehreren SCs in einem gleichen Cluster gruppiert werden, und wobei die Distanzmatrix errechnet wird auf Basis der folgenden Formel (I),

$$D_d (RSC, SC_m) = 1 - (2^* N_{(total)} / [N_{(CTIR)} + N_{(RTIR)}])$$

wobei $D_d (RSC, SC_m)$ die Distanzfunktion zwischen dem RSC-Genom und einem entsprechenden $SC_m$-Genom repräsentiert, wobei $N_{(total)}$ die Anzahl der Insertionsregionen, welche sowohl im RSC-Genom als auch im $SC_m$-Genom anwesend sind, ist, wobei $N_{(CTIR)}$ die Gesamtanzahl der Insertionsregionen, welche im $SC_m$-Genom anwesend ist, darstellt und $N_{(RTIR)}$ die Gesamtanzahl der Insertionsregionen, welche im RSC-Genom anwesend ist, darstellt; wobei $D_d (RSC, SC_m)$ die Distanz repräsentiert, auf einer Skala von 0 bis 1, wobei eine Distanz von 0 die klonale Identität zwischen dem RSC und dem entsprechenden $SC_m$ repräsentiert und 1 einen klonalen Unterschied repräsentiert.

2. Verfahren gemäß Anspruch 1, wobei die Paired-End-Sequenzierung eine Sequenzierung eines gegebenen Nucleinsäuremoleküls von beiden Enden des Nucleinsäuremoleküls involviert, wobei Paare von Sequenzabschnitten für ein gegebenes Nucleinsäuremolekül erzeugt werden, welche ein Fragment des zu sequenzierenden Genoms repräsentieren.

3. Verfahren gemäß Anspruch 1 oder 2, wobei der RSC mit einer höheren Sequenzabdeckung im Vergleich zu dem einen oder den mehreren SCs sequenziert wird.

4. Verfahren gemäß einem der obigen Ansprüche, wobei die MCB aus der Insertion des Transgens an verschiedenen Positionen in das HPC-Genom resultiert, wobei die willkürliche Insertion präferentiell unter Verwendung eines retroviralen Vektors durchgeführt wird.

5. Verfahren gemäß einem der bisherigen Ansprüche, wobei die Bestimmung der TIRs umfasst die Klassifikation der Paired-End-Sequenzabschnitt-1-Sequenzen und der Paired-End-Sequenzabschnitt-2-Sequenzen, abgeleitet aus Paired-End-Bibliotheken in 4 Klassen, wobei

• Klasse 1 Sequenzabschnitt-1-Sequenzen umfasst, welche mit dem Transgen korrelieren;
• Klasse 2 Sequenzabschnitt-2-Sequenzen umfasst, welche mit dem HPC-Genom korrelieren;
• Klasse 3 Sequenzabschnitt-2-Sequenzen umfasst, welche mit dem Transgen korrelieren; und
• Klasse 4 Sequenzabschnitt-2-Sequenzen umfasst, welche mit dem HPC-Genom korrelieren;

wobei der Sequenzabschnitt 1 und der Sequenzabschnitt 2 entsprechende Vorwärts- und Rückwärts-Sequenzabschnitte repräsentieren, welche mit den 5'- und 3'-Enden eines gegebenen Nucleinsäuremoleküls innerhalb eines Nucleinsäureclusters, welcher bei der Sequenzierung erstellt wurde, aus einer Nucleinsäurebibliothek des RSC oder des einen oder mehreren SCs korrespondiert.

6. Verfahren gemäß Anspruch 5, wobei Sequenzabschnitt-1-Sequenzen kombiniert werden mit den entsprechenden Sequenzabschnitt-2-Sequenzen unter Verwendung eines Fließzellsequenzidentifizierers, wobei der Sequenzidentifizierer umfasst: eine Information der Fließzell-Spur, die Kachelnummer innerhalb der Fließzelle, die "X"-Koordinate des Nucleinsäureclusters innerhalb der Kachel und die "Y"-Koordinate des Nucleinsäureclusters innerhalb der Kachel, wodurch jedes Sequenzpaar, welches mit Sequenzabschnitt 1 und Sequenzabschnitt 2 korrespondiert, einer eindeutigen Position innerhalb der Fließzelle zugeordnet wird.

7. Verfahren gemäß Anspruch 5 und 6, wobei die entsprechenden Sequenzabschnitt-1- und Sequenzabschnitt-2-Sequenzen eines entsprechenden Sequenzabschnitts separat mit den bekannten Sequenzen des Transgens und des HPC-Genoms aligned werden (vergleichend angeordnet werden).

8. Verfahren gemäß einem der Ansprüche 5 bis 7, wobei nur die Sequenzabschnittpaare, welche Klasse-1- und Klasse-4-Sequenzen umfassen, und die Sequenzabschnittpaare, welche Klasse-2- und Klasse-3-Sequenzen umfassen, für die weitere Analyse ausgewählt werden.

9. Verfahren gemäß einem der Ansprüche 5 bis 8, wobei die TIRs durch das Alignment der Paired-End-Sequenz-Abschnittssequenzen, welche der Klasse 2 und Klasse 4 entsprechen, mit dem HPC-Genom identifiziert werden, wodurch eine 2kb-Region für jede der TIRs im HPC-Genom definiert wird.

10. Verfahren gemäß einem der vorherigen Ansprüche, umfassend die Bestimmung von n RTIRs mit der höchsten Sequenzabdeckung in der Paired-End-NGS-Bibliothek, wobei n eine ganze Zahl von 5 bis 50 ist, vorzugsweise 5, 10, 15, 20, 25, 30, 35, 40, 45 oder 50.

11. Verfahren gemäß Anspruch 10, wobei die ersten n RTIRs mit der höchsten Sequenzabdeckung bestimmt werden auf Basis

a) der Anzahl der Sequenzabschnitte einer entsprechenden Paired-End-Sequenzabschnittsequenz entsprechend Klasse 2 und 4, welche mit dem HPC-Genom korrelieren, wobei die höhere Anzahl von Sequenzabschnitten einen Einschluss als RTIR anzeigt; und
b) des Teilüberlapps der Anzahl von Sequenzabschnitten einer entsprechenden Paired-End-Sequenzabschnittsequenz entsprechend Klasse 2 und 4, wobei der niedrigere Teilüberlapp einer Anzahl von Sequenzabschnitten den Einschluss als ein RTIR anzeigt.

12. Verfahren gemäß Anspruch 10 oder 11, wobei jede der ersten n RTIRs im RSC-Genom verglichen wird mit der entsprechenden genomischen Lokalisation der CTIRs in jedem des einen SC-Genoms oder der mehreren SC-Genome.

13. Verfahren gemäß Anspruch 12, wobei der Vergleich der RTIRs in dem RSC und der CTIRs in dem einen SC oder den mehreren SCs erreicht wird durch die Herstellung einer Anwesenheits-/Abwesenheits-Matrix von Insertionsregionen, wobei eine Matrixdimension die n RTIRs des Transgens im RSC-Genom repräsentiert und eine andere, vorzugsweise orthogonale Matrixdimension den RSC und jeden des einen SC oder der mehreren SCs repräsentiert.

14. Verfahren gemäß Anspruch 13, wobei die Anwesenheit oder Abwesenheit einer entsprechenden CTIR in dem einen SC oder den mehreren SCs im Verhältnis zu einer entsprechenden RTIR in dem RSC repräsentiert wird in der Matrix als ein binärer Farbcode, wobei die erste Farbe die entsprechende Anwesenheit oder Abwesenheit einer entsprechenden RTIR im RSC repräsentiert, die entsprechende Anwesenheit oder Abwesenheit einer entsprechenden CTIR in dem einen SC oder den mehreren SCs und wobei eine zweite Farbe die entsprechende Abwesenheit oder Anwesenheit einer entsprechenden RTIR im RSC, die entsprechende Abwesenheit oder Anwesenheit einer entsprechenden CTIR in dem einen SC oder den mehreren SCs repräsentiert.

15. Verfahren gemäß einem der vorherigen Ansprüche, wobei die Parameter $N_{(total)}$, $N_{(CTIR)}$ und/oder $N_{(RTIR)}$ berechnet werden auf Basis der Anwesenheits-/Abwesenheits-Matrix der Insertionsregionen, welche gemäß einem der Ansprüche 1 oder 14 erstellt wurde.

16. Verfahren gemäß einem der vorherigen Ansprüche, wobei das Verfahren die Repräsentierung des einen SC oder der mehreren SCs im Verhältnis zu den RSC auf einer allgemeinen Distanzmatrix umfasst.

17. Verfahren gemäß Anspruch 16, wobei zwei entsprechende Genome als gemeinsamer Cluster verstanden werden, wenn die Distanz zwischen ihnen, die gemäß Formel (I) errechnet wurde, 0 ist.

**Revendications**

1. Procédé de détermination de la clonalité d'une BCM (banque cellulaire maître) laquelle BCM est le résultat de l'insertion, prévisible ou non prévisible, d'un transgène de séquence connue dans un génome de CPH (cellule progénitrice hôte) de séquence connue, lequel procédé comporte les étapes suivantes :

   a) identifier une ou plusieurs RIT (région(s) d'insertion de transgène) dans le génome d'une cellule de SCR (sous-clone de référence), lequel SCR a été isolé à partir de la BCM dont on veut déterminer la clonalité, laquelle identification est réalisée par :

      i) séquençage à extrémités appariées dudit génome de SCR, pour obtenir une séquence de génome de SCR ou des séquences de génome de SCR,
      ii) et alignement par superposition de cette ou ces séquence(s) de génome de SCR à ladite séquence connue de génome de CPH et à ladite séquence connue de transgène,

   ce qui fournit une ou plusieurs RIT (région(s) d'insertion de transgène) ;
   b) déterminer une ou plusieurs RIT, identifiée(s) dans l'étape (a),
   qui présentent le degré de recouvrement de séquences le plus élevé, étant entendu que ledit « recouvrement de séquences » renvoie au nombre de fois qu'une séquence donnée d'acide nucléique contenant une RIT donnée est lue, au cours du processus de séquençage, par lectures de séquences en chevauchement partiel, ladite ou lesdites RIT présentant le degré de recouvrement de séquence le plus élevé étant alors désignée(s) comme RITR (RIT de référence) ;
   c) identifier une ou plusieurs RIT (région(s) d'insertion de transgène) dans le ou les génome(s) respectif(s) d'un ou de plusieurs SC (sous-clone(s)),
   étant entendu que chacun de ces SC a été isolé à partir de la BCM dont on veut déterminer la clonalité, mais est indépendant dudit SCR,
   laquelle identification est réalisée comme suit :

      i) séquençage à extrémités appariées de chaque génome respectif de SC, pour obtenir une séquence de génome de SC ou des séquences de génome de SC,
      ii) et alignement par superposition de la séquence ou de chacune des séquences respectives de génome de SC à ladite séquence connue de génome de CPH et à ladite séquence connue de transgène,

   ce qui fournit une ou plusieurs RITC (région(s) d'insertion de transgène à comparer) ;
   d) comparer ladite ou lesdites RITR déterminées dans l'étape (b) avec les RITC respectives déterminées dans l'étape (c) ;
   e) évaluer la correspondance entre ladite ou chacune desdites RITC présentes dans un SC respectif et les RITR correspondantes présentes dans ledit SCR, étant entendu qu'on évalue la relation entre ledit SCR et ledit ou chacun desdits SC en calculant une matrice de distance ;
   f) et déterminer la clonalité de ladite BCM en se basant sur ladite correspondance évaluée dans l'étape (e),
   étant entendu que la BCM est considérée comme étant monoclonale si ledit SCR et ledit ou lesdits SC sont regroupés au sein du même cluster, et étant entendu qu'on calcule la matrice de distance en appliquant la formule (I) suivante :

$$D_d(SCR, SC_m) = 1 - [2.N_{(total)}/(N_{(RITC)} + N_{(RITR)})]$$

   dans laquelle

      - $D_d(SCR, SC_m)$ représente la fonction distance entre ledit génome de SCR et un génome de $SC_m$ respectif,
      - $N_{(total)}$ représente le nombre de régions d'insertion présentes à la fois dans ledit génome de SCR et dans ledit génome de $SC_m$ respectif,

- $N_{(RITC)}$ représente le nombre de régions d'insertion présentes dans ledit génome de $SC_m$ respectif,
- et $N_{(RITR)}$ représente le nombre de régions d'insertion présentes dans ledit génome de SCR,

et étant entendu que $D_d(SCR, SC_m)$ représente la distance sur une échelle allant de 0 à 1, où une distance de 0 indique une identité de clones entre ledit SCR et un $SC_m$ respectif, et une distance de 1 indique une différence entre clones.

2. Procédé conforme à la revendication 1, dans lequel le séquençage à extrémités appariées implique le séquençage d'une molécule donnée d'acide nucléique à partir des deux extrémités de cette molécule d'acide nucléique, ce qui génère des paires de lectures pour une molécule d'acide nucléique donnée représentant un fragment du génome à séquencer.

3. Procédé conforme à la revendication 1 ou 2, dans lequel le génome dudit SCR est séquencé avec un plus grand recouvrement de séquences, par comparaison avec ledit ou lesdits SC.

4. Procédé conforme à l'une des revendications précédentes, dans lequel ladite BCM est le résultat de l'insertion dudit transgène en de multiples positions dans ledit génome de CPH, cette insertion aléatoire étant de préférence effectuée au moyen d'un vecteur rétroviral.

5. Procédé conforme à l'une des revendications précédentes, dans lequel la détermination des RIT comporte le fait de répartir les séquences de première lecture à extrémités appariées et les séquences de deuxième lecture à extrémités appariées dérivées de bibliothèques de lectures à extrémités appariées en quatre classes, à savoir :

   - une classe 1 qui comprend les séquences de première lecture qui coïncident avec celle dudit transgène,
   - une classe 2 qui comprend les séquences de première lecture qui coïncident avec celle dudit génome de CPH,
   - une classe 3 qui comprend les séquences de deuxième lecture qui coïncident avec celle dudit transgène,
   - une classe 4 qui comprend les séquences de deuxième lecture qui coïncident avec celle dudit génome de CPH,

   étant entendu que ladite première lecture et ladite deuxième lecture représentent respectivement la lecture « en avant » et la lecture « en arrière », correspondant aux extrémités 5' et 3' d'une molécule donnée d'acide nucléique au sein d'un cluster d'acide nucléique, générées lors du séquençage d'une bibliothèque d'acide nucléique dudit SCR ou dudit ou desdits SC.

6. Procédé conforme à la revendication 5, dans lequel les séquences de première lecture sont combinées avec les séquences de deuxième lecture correspondantes au moyen d'un dispositif d'identification de séquences à cellule à flux continu, lequel dispositif d'identification de séquences comprend les informations portant sur la piste de la cellule à flux continu, le nombre de cases dans la cellule à flux continu, la coordonnée « x » du cluster d'acide nucléique à l'intérieur d'une case et la coordonnée « y » du cluster d'acide nucléique à l'intérieur d'une case, ce qui permet d'attribuer à chaque paire de séquences correspondant aux séquences de première lecture et de deuxième lecture une position unique dans la cellule à flux continu.

7. Procédé conforme à l'une des revendications 5 et 6, dans lequel les séquences de première lecture et de deuxième lecture respectives d'une paire respective de lectures sont séparément alignées par superposition aux séquences connues du transgène et du génome de CPH.

8. Procédé conforme à l'une des revendications 5 à 7, dans lequel seules les paires de lectures comprenant des séquences de classe 1 et de classe 4 et les paires de lectures comprenant des séquences de classe 2 et de classe 3 sont sélectionnées pour la suite des analyses.

9. Procédé conforme à l'une des revendications 5 à 8, dans lequel on identifie lesdites RIT en alignant par superposition les séquences de lectures à extrémités appariées correspondant à la classe 2 et à la classe 4 sur le génome de CPH, ce qui permet de définir, pour chacune desdites RIT, une région de 2 kb dans le génome de CPH.

10. Procédé conforme à l'une des revendications précédentes, qui comporte le fait de déterminer un nombre N de RITR dotées du recouvrement de séquence le plus élevé dans la bibliothèque pour SPG (séquençage de prochaine génération) à extrémités appariées, étant entendu que N est un nombre entier qui vaut de 5 à 50 et de préférence 5, 10, 15, 20, 25, 30, 35, 40, 45 ou 50.

**11.** Procédé conforme à la revendication 10, dans lequel on détermine les premières N RITR dotées du recouvrement de séquence le plus élevé en se basant sur :

a) le nombre de lectures d'une séquence de lecture à extrémités appariées respective correspondant à la classe 2 et à la classe 4 coïncidant avec le génome de CPH, étant entendu qu'un nombre élevé de lectures est une indication pour l'inclusion en tant que RITR,

b) et le chevauchement partiel des lectures d'une séquence de lecture à extrémités appariées respective correspondant à la classe 2 et à la classe 4, étant entendu qu'un faible chevauchement partiel de ces lectures est une indication pour l'inclusion en tant que RITR.

**12.** Procédé conforme à la revendication 10 ou 11, dans lequel chacune des premières N RITR présentes dans ledit génome de SCR est comparée avec l'endroit du génome correspondant desdites RITC dans chacun dudit ou desdits génomes de SC.

**13.** Procédé conforme à la revendication 12, dans lequel on réalise la comparaison desdites RITR dans ledit SCR et desdites RITC dans ledit ou lesdits SC en générant une matrice de présence/absence des régions d'insertion, étant entendu qu'une dimension de la matrice représente lesdites N RITR dudit transgène dans ledit génome de SCR, et qu'une autre dimension, de préférence orthogonale, de la matrice représente ledit SCR et ledit SC ou chacun desdits SC.

**14.** Procédé conforme à la revendication 13, dans lequel la présence ou l'absence d'une RITC respective dans ledit ou lesdits SC, par rapport à une RITR respective dans ledit SCR, est représentée dans la matrice au moyen d'un code de couleurs binaire, dans lequel une première couleur représente la respective présence ou absence d'une RITR respective dans ledit SCR, la respective présence ou absence d'une RITC respective dans ledit ou lesdits SC, et une deuxième couleur représente la respective présence ou absence d'une RITR respective dans ledit SCR, la respective présence ou absence d'une RITC respective dans ledit ou lesdits SC.

**15.** Procédé conforme à l'une des revendications précédentes, dans lequel on calcule les paramètres $N_{(total)}$, $N_{(RITC)}$ et/ou $N_{(RITR)}$ en se basant sur la matrice de présence/absence des régions d'insertion générées selon l'une ou l'autre des revendications 1 et 14.

**16.** Procédé conforme à l'une des revendications précédentes, lequel procédé comporte le fait de représenter ledit ou lesdits SC, par rapport au SCR, sur une matrice de distance commune.

**17.** Procédé conforme à la revendication 16, dans lequel deux génomes respectifs sont considérés comme appartenant à un cluster commun si la distance entre eux calculée au moyen de la formule (I) est nulle.

Figure 1 (a)

Figure 1 (b)

Figure 1 (c)

Figure 2

Figure 3

EP 3 384 045 B1

# Concept of determination of RTIRs in RSC

First 5 reference transgene insertion regions (RTIRs):

$$RTIR_2 > RTIR_5 > RTIR_8 > RTIR_6 > RTIR_4$$

Figure 4

EP 3 384 045 B1

Figure 5

Formula (I):

$$D_d(A, B) = 1 - \left[ \frac{2 * N_{(total)}}{(N_{(A)} + N_{(B)})} \right]$$

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **SUFFICOOL et al.** *J. Am. Acad. Derm.,* August 2015, vol. 73 (2), 228-36
- **EWING B ; GREEN P.** Base-calling of automated sequencer traces using phred. II. Error probabilities. *Genome Res.,* 1998, vol. 8, 186-194
- Mammalian cel culture for biopharamcutical production. **JINYOU ZHANG.** Manual of industrial microbiology and biotechnology
- **BOEGER et al.** Structural basis of eukaryotic gene expression. *FEBS Lett,* 2005, vol. 579, 899-903
- **KIM et al.** CHO cells in biotechnology for production of recombinant proteins: Current state and further potential. *Appl Microbiol Biotechnol,* 2012, vol. 93, 917-30
- **GHADERI et al.** Production platforms for biotherapeutic glycoproteins. Occurrence, impact, and challenges of non-human sialylation. *Biotechnol Genet Eng Rev,* 2012, vol. 28, 147-75
- GPEx®: a flexible method for the rapid generation of stable, high expressing, antibody producing mammalian cell lines. **GREGORY T. BECK.** Current Trends in Monoclonal Antibody Development and Manufacturing. Springer, 2010
- **LI et al.** Fast and accurate short read alignment with Burrows Wheeler transform. *Bioinformatics,* 2009, vol. 25 (14), 1754-60
- Sage University Paper series on Quantitative Application in the Social Sciences. **KRUSKAL ; WISH.** Multidimensional Scaling. Sage Publications, 1978, vol. 07-011
- **MICHAEL R. ANDERBERG.** Cluster analysis for applications. Academic Press, 1973
- **BUSHMAN et al.** Genome-wide analysis of retroviral DNA integration. *Nat Rev Microbiol,* 2005, vol. 3 (11), 848-858
- **FELICE et al.** Transcription factor binding sites are genetic determinants of retroviral integration in the human genome. *PLoS ONE,* 2009, vol. 4 (2), e4571
- **LAFAVEY et al.** MLV integration site selection is driven by strong enhancers and active promoters. *Nucleic Acids Research,* 2014, vol. 42 (7), 4257-4269